# EUROPEAN PATENT SPECIFICATION

(11) **EP 2 059 527 B1**
(45) Date of publication and mention of the grant of the patent: **03.12.2014**
(21) Application number: 07803166.3
(22) Date of filing: 03.09.2007
(51) Int. Cl.: C07K 14/435, A61K 47/48, A61K 38/00

(54) **MODIFIED GLYCOPROTEINS**
MODIFIZIERTE GLYKOPROTEINE
GLYCOPROTÉINES MODIFIÉES

(30) Priority: 01.09.2006 EP 06120000; 02.02.2007 EP 07101674
(43) Date of publication of application: 20.05.2009
(73) Proprietor: Novo Nordisk Health Care AG, Thurgauerstrasse 36/38 8050 Zürich (CH)
(72) Inventor: BEHRENS, Carsten, 2200 Copenhagen N. (DK)
(74) Representative: Nilsson, Karin Norvin
(86) International application number: PCT/EP2007/059180
(87) International publication number: WO 2008/025856

(56) References cited:
- EP-A2- 0 605 963
- WO-A-92/16555
- WO-A-2004/000366
- WO-A2-2005/014024
- US-A1- 2005 220 762
- DENNIS M S ET AL: "Albumin binding as a general strategy for improving the pharmacokinetics of proteins" JOURNAL OF BIOLOGICAL CHEMISTRY, AMERICAN SOCIETY OF BIOLOCHEMICAL BIOLOGISTS, BIRMINGHAM,, US, vol. 277, no. 38, 20 September 2002 (2002-09-20), pages 35035-35043, XP002285300 ISSN: 0021-9258 cited in the application

## Description

### FIELD OF THE INVENTION

The present invention relates to the preparation of improved drugs, especially to the preparation of modified glycoproteins having improved pharmacodynamic and/or pharmacokinetic properties.

### BACKGROUND OF THE INVENTION

Proteins of biological origin hold great promise as therapeutical agents as they often possess high efficacy and high selectivity towards their natural ligands. Being of biological origin increases the likelihood that they are non-toxic and thus safer to use than conventional small molecular drugs, as the organism already posses well defined clearing mechanisms as well as metabolic pathways for their disposal. This in combination with the fact, that proteins now can be produced by recombinant DNA techniques in a variety of different expression systems, allowing for large-scale production, render proteins ideal drug candidates. However, therapeutically interesting proteins such as hormones, soluble receptors, cytokines, enzymes, etc., often have short circulation half-life in the body, generally reducing their therapeutic utility.

Therapeutic proteins are removed from circulation by a number of routes. For some pharmacologically active proteins, there are specific receptors which mediate removal from circulation. Proteins which are glycosylated may be cleared by lectin-like receptors in the liver, which exhibit specificity only for the carbohydrate portion of those molecules. Nonspecific clearance by the kidney of proteins and peptides (particularly non-glycosylated proteins and peptides) below about 50 kDa has also been documented. It has been noted that asialo-glycoproteins are cleared more quickly by the liver than native glycoproteins or proteins lacking glycosylation (Bocci (1990) Advanced Drug Delivery Reviews 4: 149). Therapeutic proteins are also cleared from circulation by the immune system in the event that they are not completely identical to autologous proteins, since even small variations in amino acid sequence or 3-dimensional structure can render a therapeutic protein immunogenic. The immune response induced by a therapeutic protein can further have various undesired effects apart from the accelerated removal from circulation: Antibodies may interfere with or block the therapeutic effect via steric hindrance of access to binding sites in the therapeutic protein, induced antibodies may cross-react with autologous proteins and thereby result in autoimmune reactions etc. It is also of interest to modify therapeutic proteins so as to target them to certain cells, organs or tissues. Conjugation or fusion of proteins to ligand molecules that have high affinity for molecules present in specialised cells or tissues is one known way of achieving this effect.

EP0605963 discloses the preparation of polymer modified glycoproteins, wherein the bond between the polymer and the glycoprotein is either a hydrazone or an oxime, obtained by reacting an aldehyde from the glycoprotein with a polymer having either a hydrazine or oxyiamine reactive group.

WO2005014024 provides conjugates comprising a polymer and a protein linked via an oxime bond.

There is therefore a general need for provision of methods of preparing modified (therapeutic) proteins which exhibit prolonged serum half-life and/or reduced immunogenicity and/or improved pharmacological properties.

### SUMMARY OF THE INVENTION

The present invention provides for the prolongation of the circulating half-life of soluble glycoprotein derivatives, thus reducing the quantity of injected material and frequency of injection required for maintenance of therapeutically effective levels of circulating glycoprotein for treatment or prophylaxis. The short *in vivo* plasma half-life of certain therapeutically active glycoproteins is undesirable due to the frequency and the amount of soluble protein which would be required in treatment or prophylaxis. The present invention provides means to prolong the circulating half-life of such glycoproteins with an effective change to the glycoprotein structure and with the substantial maintenance of biological activity.

The present invention provides for convenient methods of preparing glycoprotein derivatives, where an oxime of a polymeric moiety is introduced at a glycosyl group in the glycoprotein, where said modified glycoprotein has improved pharmacologic properties compared to the starting glycoprotein.

Thus, the invention relates to a method for preparing a modified glycoprotein with the general structure

(M-L-O-N=CH)ₙ-P-(CH=N-O-L'-M')ₘ (formula I)

wherein M and optionally M' independently is a polymeric moiety for increasing the molecular weight of the modified glycoprotein, and wherein L and L' independently represent a bivalent linker, and wherein P represents a glycoprotein comprising one or more oxidized glucan terminals of said glycoprotein, O, N, C and H represents oxygen, nitrogen, carbon and hydrogen atoms respectively, n is 1-10, m is 0-50, the method comprising the steps of
a) oxidizing with periodate ions at least one glycan terminal present on glycoprotein P*, wherein P* represents a plurality of glycoforms to obtain the glycoprotein P(CHO)ₙ₊ₘ containing one or more aldehyde groups, and
b) reacting P(CHO)ₙ₊ₘ with M-L-O-NH₂ to obtain the modified glycoprotein with the structure (M-L-O-N=CH)ₙ-P-(CHO)ₘ, and
c) reacting any non-reacted aldehyde group in glycoprotein with structure (M-L-O-N=CH)ₙ-P-(CHO)ₘ with M'-L'-O-NH₂ to obtain the modified glycoprotein with the structure (M-L-ON=CH)ₙ-P-(CH=N-O-L'-M')ₘ,
wherein said periodate ions are present in an amount of 0.1-5 equivalents relative to the number of non-reducing glycan terminals present on the glycoprotein, and wherein said modified glycoprotein has improved pharmacologic properties compared to the starting glycoprotein P* and has retained its functional activity.

It is to be understood that the aldehyde group or groups in P-(CHO)ₙ₊ₘ may transiently exist in its/their geminal diol form(s), or as hemiacetal(s).
Also it is to be understood by P- and -P- that the glycoprotein comprising one or more oxidized glucan terminals of said glycoprotein, wherein the chemical bonds is to said one or more glucan terminals.

### DETAILED DESCRIPTION OF THE INVENTION.

It is to be understood by "a plurality of modified glycoproteins" that individual modified glycoprotein molecules within a preparation may not have the exact same chemical structure, which may vary considerably within individual glycoprotein molecules. The starting glycoprotein P* typically exists in different glycoforms. By "glycoforms" is meant protein isoforms of the same protein having different polysaccharides attached to them, by either posttranslational or cotranslational modifications. Also some molecules with a preparation may not by modified at all, and some molecules may be modified with more than one polymeric moiety, which again may be modified on different glycan terminals of each individual glycoprotein. P* is therefore to be considered as a plurality of protein P glycoforms.

The terms "non-reducing glycan terminal" or "non-reducing glycan terminals" refers to the terminal end or ends of an oligosaccharide that is not reduced using e.g. Fehling solution or Tollens reagens, in contrast to the reducing end of an oligosaccharide, that are oxidized with these reagents. In glycoproteins, oligosaccharides are usually attatched to the protein at their reducing terminal via a glycoside- or glycosamino bond. Non-reducing ends of the oligosaccharide include by illustration and not by limitation terminal sialic acids residues on complex N- and O-glycans; terminal sialic acids residues on hybride N-glycans; terminal mannose residues on high-mannose N-glycans; terminal galactose residues on complex N-and O-glycans; terminal galactose residues on hybride N-glycans; terminal N-acetylgalactose residues on complex N-and O-glycans; terminal N-acetylgalactose residues on hybride N-glycans; terminal N-acetylglucosamine residues on complex N-and O-glycans; terminal N-acetylglucosamine residues on hybride N-glycans; terminal mannose residues on full or partly exposed trimannose core residues; terminal fucose residues, including core fucose residues; terminal glucose and xylose residues.

The starting glycoprotein P* may optionally be trimmed with sialidases, galactosidases, N-acetylglucosaminidases, mannosidases or fucosidases, if a particular terminal glycan moiety is prefered for the periodate reaction. Alternatively the glycan moiety may be re-modelled using e.g. sialyltransferase, galactosyltransferases, N-acetylglucosaminosyltransferases; N-acetylgalactosaminosyltransferases; mannosyltransferases and respective sugar donors such as CMP-Sia; UDP-Gal; GDP-Fuc ect.

The use of periodate to oxidize glycan terminals of glycoproteins is well documented and has been used as a general method for preparing protein conjugates. International patent application with publication number WO 06/071801 describes the oxidation of glycan terminals on von Willebrand Factor and its application for preparing pegylated versions of the protein. International patent application with publication number WO 00/23114 describes methodology for preparing pegylated versions of interferon-beta-1a, and International patent application with publication number WO 92/16555 describes several methods to conjugate PEG moieties to proteins, in which formation of aldehyde functions on glycan terminals using periodate is mentioned.

Besides cleaving diols on sugar moieties, periodate is also known to oxidize amino acid side chains such as metheonine, and to cleave N-terminal amino acids containing amino alcohols moieties such as serine and threonine. In some cases, side chain oxidation of metheonine may lead to changes in the biological profile of the protein and in particular to changes in the biological activity. As stated in Kornfelt, T; Persson, E. and Palm, L.; Archives of Biochemistry and Biophysics Vol. 363 , No. 1 pp. 43-54 (1999 ), the activated form of recombinant coagulation factor FVII (e.g. FVIIa) has proven to be highly sensitive towards metheonine oxidation. After oxidation of metheonine 298 and 306 with hydrogen peroxide, FVIIa binding to soluble tissue factor (TF) is weaker, as manifested by a threefold increase in the dissociation constant. Also the amidolytic activity in the metheonine oxidized FVIIa in complex with soluble TF is only 80% compared to that of the native FVIIa-TF complex.

Not surprisingly, and in line with these observations, we have found that when using periodate mediated conjugation methods as those generally described in the litteratur and mentioned above, a significant and in some cases complete loss in the peptidolytical activity of FVIIa, as measured by its ability to cleave known peptide substrates, is observed.
Thus the known methods for periodate mediated conjugation of e.g. PEG moieties is of very limited use, when working with glycoproteins that are highly sensitive to oxidation.
This present invention describes general methods for circumventing this problem and thus discloses for the first time methods that enable the preparation of oxime conjugated glycoproteins with retained functional activities.

When applying low concentrations, and in term of equivalents - near stoichiometric amount of periodate relative to the number of non-reducing glycan terminals present on the glycoprotein, biological activity, can be preserved. Surprisingly, oxidation with stoichiometric amount of periodate-ions and subsequent conjugation chemistry procced within acceptable time frames, and provide biological functional protein conjugates in high purity and with moderate to good yields.

In one embodiment the present invention relates to a method for preparing modified glycoprotein, wherein said glycoprotein is N-glycosylated and/or O-glycosylated and/or contains sialic acid moieties.

In a further embodiment the present invention relates to a method for preparing modified glycoprotein, which method comprises the further step of confirming that the modified glycoprotein has improved pharmacologic properties compared to the starting glycoprotein.

In one embodiment the improved pharmacologic property is selected from the group consisting of increased bioavailability, increased functional *in vivo* half-life, increased *in vivo* plasma half-life, reduced immunogenicity, increased protease resistance, increased affinity for albumin, improved affinity for a receptor, increased storage stability, decreased functional *in vivo* half-life, decreased *in vivo* plasma half-life.

In one embodiment the increased half-life is obtained by M and/or M' being a group that increases molecular weight so that renal clearance is reduced or abolished and/or by M and/or M' being a group that masks binding partners for hepatic receptors.

In one embodiment the reduced immunogenicity is obtained by M and/or M' being a group which blocks antibody binding to immunogenic sites.

In one embodiment the improved affinity for albumin is obtained by M and/or M' being a group which has high affinity for albumin.

In one embodiment the improved affinity for a receptor is obtained by M and/or M' being a group which specifically binds a surface receptor on a target cell.

In a further embodiment the present invention relates to a method for preparing modified glycoprotein with the general structure

(M-L-O-N=CH)ₙ-P-(CH=N-O-L'-M')ₘ (formula I),

wherein M and/or M' is selected from the group consisting of: a low molecular weight organic charged radical, which may contain one or more carboxylic acids, amines, sulfonic acids, phosphonic acids, or combinations thereof; a low molecular weight neutral hydrophilic molecule, such as cyclodextrin or optionally a branched polyethylene chain; a low molecular weight hydrophobic molecule such as a fatty acid or cholic acid or derivatives thereof; a polyethylene glycol with an average molecular weight of 2-40 kDa; a well-defined precision polymer such as a dendrimer with an exact molecular mass ranging from 700 Da to 20 kDa; a substantially non-immunogenic polypeptide such as albumin, an antibody or a part of an antibody optionally containing a Fc-domain; and a high molecular weight organic polymer.

In a further embodiment the present invention relates to a method for preparing modified glycoprotein with the general structure

(M-L-O-N=CH)ₙ-P-(CH=N-O-L'-M')ₘ (formula I),

wherein M and/or M' is selected from the group consisting of a dendrimer, polyalkylene oxide (PAO), including polyalkylene glycol (PAG), such as polyethylene glycol (PEG) and polypropylene glycol (PPG), branched PEG, polyvinyl alcohol (PVA), polycarboxylate, polyvinylpyrolidone, polyethylene-co-maleic acid anhydride, polystyrene-co-maleic acid anhydride, dextran, carboxymethyl-dextran.

In a further embodiment, M and/or M' is selected from hydroxyalkyl starch (HAS) and hydroxyethyl starch (HES) such as the compounds discribed in Clin Pharmacokinet 2005; 44 (7): 681-699, and disclosed in WO2006094810A2; Suitable activated forms of HES is disclosed in WO2005092369A2.

In a further embodiment, M and/or M' is poly(1-hydroxymethylethylene hydroxymethylformal) (PHF) or similar degraded dextranes such as the ones described in WO2006094810A2, where also suitable activated forms of the polymers are disclosed.

In a further embodiment, M and/or M' is selected from zwitter ionic polymers such as those disclosed in WO03062290A1. In a specific embodiment, the polymer is 2-methacryloyloxy-2'-ethyltrimethylammoniumphosphate inner salt (MPC).

In a further embodiment the present invention relates to a method for preparing modified glycoprotein with the general structure

(M-L-O-N=CH)ₙ-P-(CH=N-O-L'-M')ₘ (formula I),

wherein M and/or M' is selected from the group consisting of a serum protein binding-ligand and a small organic molecule containing moieties that under physiological conditions alters charge properties, a structure which inhibits glycans from binding to receptors, and a neutral substituent that prevent glycan specific recognition.

In a further embodiment the present invention relates to a method for preparing modified glycoprotein with the general structure

(M-L-O-N=CH)ₙ-P-(CH=N-O-L'-M')ₘ (formula I),

wherein P is selected from FVII, FVIII, FIX, FX, FII, FV, protein C, protein S, tPA, PAI-1, tissue factor, FXI, FXII, FXIII, as well as sequence variants thereof; immunoglobulins, cytokines such as interleukins, alpha-, beta-, and gamma-interferons, colony stimulating factors including granulocyte colony stimulating factors, fibroblast growth factors, platelet derived growth factors, phospholipase-activating protein (PUP), insulin, plant proteins such as lectins and ricins, tumor necrosis factors and related alleles, soluble forms of tumor necrosis factor receptors, interleukin receptors and soluble forms of interleukin receptors, growth factors such as tissue growth factors, such as TGFa's or TGFps and epidermal growth factors, hormones, somatomedins, erythropoietin, pigmentary hormones, hypothalamic releasing factors, antidiuretic hormones, prolactin, chorionic gonadotropin, follicle-stimulating hormone, thyroid-stimulating hormone, tissue plasminogen activator, and immunoglobulins such as IgG, IgE, IgM, IgA, and IgD, and fragments thereof, or any fusion proteins comprising any of the above mentioned proteins or fragments thereof.

In a further embodiment the present invention relates to a method for preparing modified glycoprotein with the general structure

(M-L-O-N=CH)ₙ-P-(CH=N-O-L'-M')ₘ (formula I),

wherein the glycoprotein is a Factor VII polypeptide.

In a further embodiment the present invention relates to a method for preparing modified glycoprotein with the general structure

(M-L-O-N=CH)ₙ-P-(CH=N-O-L'-M')ₘ (formula I),

wherein the glycoprotein has the amino acid sequence of wild-type human Factor VII.

In a further embodiment the present invention relates to a method for preparing modified glycoprotein with the general structure

(M-L-O-N=CH)ₙ-P-(CH=N-O-L'-M')ₘ (formula I),

wherein the glycoprotein is a Factor VIII polypeptide.

"Factor VIII" or "FVIII polypeptide", as used herein includes FVIII:C, B-domain deleted versions of FVIII, amino acid variants and and combinations thereof.

In a further embodiment the present invention relates to a method for preparing modified glycoprotein with the general structure

(M-L-O-N=CH)ₙ-P-(CH=N-O-L'-M')ₘ (formula I),

wherein the modified glycoprotein exhibit at least about 10 %, such as at least about 20 %, such as at least about 40 %, such as at least about 60 %, such as at least about 80 %, such as at least about 100 % of the specific activity of un-modified Factor VII polypeptide when tested in one or more of a clotting assay, proteolysis assay, or TF binding assay as described in the present specification.

In a further embodiment the present invention relates to a method for preparing modified glycoprotein with the general structure

(M-L-O-N=CH)ₙ-P-(CH=N-O-L'-M')ₘ (formula I),

wherein the modified glycoprotein exhibits a bioavailability that is at least about 110% of the bioavailability of the un-modified glycoprotein, such as at least about 120%, about 130%, or at least about 140% of the bioavailability of the un-modified glycoprotein.

In a further embodiment the present invention relates to a method for preparing modified glycoprotein with the general structure

(M-L-O-N=CH)ₙ-P-(CH=N-O-L'-M')ₘ (formula I),

wherein the modified glycoprotein exhibits a serum half-life that is at least about 125% of the half-life of the un-modified glycoprotein, such as about 150%, about 200%, or at least about 250% of the half-life of the un-modified glycoprotein.

In a further embodiment the present invention relates to a method for preparing modified glycoprotein with the general structure

(M-L-O-N=CH)ₙ-P-(CH=N-O-L'-M')ₘ (formula I),

wherein said periodate ions are present in an amount of less than 20 equivalents, such as less than 10 equivalents, such as less than 5 equivalents, such as less than 1 equivalents relative to the number of non-reducing glycan terminals present on the glycoprotein, such as in the range of 0.1-20 equivalents, such as in the range of 0.1-10 equivalents, such as in the range of 0.1-5 equivalents, such as in the range of 0.1-1 equivalents, relative to the number of non-reducing glycan terminals present on the glycoprotein.

Thus, in one embodiment, the number of periodate ions are present in an amount of 10-20 equivalent relative to the number of non-reducing glycan terminals present on the glycoprotein. In another embodiment, the number of periodate ions are present in an amount of 5-10 equivalent relative to the number of non-reducing glycan terminals present on the glycoprotein. In another embodiment, the number of periodate ions are present in an amount of 2-5 equivalent relative to the number of non-reducing glycan terminals present on the glycoprotein. In another embodiment, the number of periodate ions are present in an amount of equivalent that substantially equals to the number of non-reducing glycan terminals present on the glycoprotein. In another embodiment, the number of periodate ions are present in an amount of 0.1-0.9 equivalent relative to the number of non-reducing glycan terminals present on the glycoprotein.

In another embodiment, a sub-stoichometric amount of periodate is used relative to the number of non reducing glycan terminals presented on the glycoprotein.
In the present specification and claims, the term "polypeptide" is a linear, singlechain molecule consisting of peptide-bonded amino acid residues. Hence, the term embraces peptides (2-10 amino acid residues), oligopeptides (11-100 amino acid residues) and proper polypeptides (in excess of 100 amino acid residues). A polypeptide is thus a structural unit, which may be biologically active, but it can also lack any function. A "protein" is in the present context a functional or non-functional molecule or complex comprising at least one polypeptide, so apart from monomers, the term also includes polymeric molecules such as homo- and heteromultimers. A protein may include prosthetic groups, and may include various glycoslylation and lipidation patterns. A "glycoprotein" as used herein is a protein that in some way is glycosylated. In some embodiments of the invention, the glycoprotein is N-glycosylated and/or O-glycosylated and/or is modified with sialic acid moeities.

In another embodiment, the method for producing the modified glycosylated molecule comprises the further step of confirming that the modified glycoprotein has improved pharmacologic properties compared to the glycosylated starting molecule.

Typically, the improved pharmacologic property is selected from the group consisting of increased bioavailability, increased functional *in vivo* half-life, increased *in vivo* plasma half-life, reduced immunogenicity, increased protease resistance, increased affinity for albumin, improved affinity for a receptor, increased storage stability.

The term "functional *in vivo* half-life" is used in its normal meaning, i.e., the time at which 50% of the biological activity of the modified glycoprotein or a reference molecule is still present in the body/target organ, or the time it takes for the activity of the modified glycoprotein or reference molecule to drop to 50% of its peak value. As an alternative to determining functional *in vivo* half-life, *"in vivo* plasma half-life" may be determined, i.e., the time at which 50% of the modified glycoproteins or reference molecules circulate in the plasma or bloodstream prior to being cleared. Determination of plasma half-life is often more simple than determining functional half-life and the magnitude of plasma half-life is usually a good indication of the magnitude of functional in vivo half-life. Alternative terms to plasma half-life include serum half-life, circulating half-life, circulatory half-life, serum clearance, plasma clearance, and clearance half-life. The functionality to be retained is normally selected from procoagulant, proteolytic, co-factor binding, receptor binding activity, or other type of biological activity associated with the particular protein.

The term "increased" as used about the functional *in vivo* half-life or plasma half-life indicates that the relevant half-life of the modified glycoprotein is statistically significantly increased relative to that of a reference molecule, such as an otherwise identical glycoprotein which has, however, not been subjected to the method of the invention. Thus, the half-life is determined under comparable conditions. For instance the relevant half-life may be increased by at least about 25%, such as by at least about 50%, e.g., by at least about 100%, 150%, 200%, 250%, or 500%. In some embodiments, the modified glycoproteins of the present invention exhibit an increase in half-life of at least about 0.25 h, preferably at least about 0.5 h, more preferably at least about 1 h, and most preferably at least about 2 h, relative to the half-life of of the un-modified glycoprotein.

Measurement of *in vivo* biological half-life can be carried out in a number of ways as described in the literature. An example using modified FVIIa (coagulation factor VIIa) of an assay for the measurement of in vivo half-life of rFVIIa and variants thereof is described in FDA reference number 96-0597. Briefly, FVIIa clotting activity is measured in plasma drawn prior to and during a 24-hour period after administration of the modified glycoprotein. The median apparent volume of distribution at steady state is measured and the median clearance determined.

"Bioavailability" refers to the proportion of an administered dose of a glycoconjugate that can be detected in plasma at predetermined times after administration. Typically, bioavailability is measured in test animals by administering a dose of between about 25-250 µg/kg of the preparation; obtaining plasma samples at predetermined times after administration; and determining the content of glycoprotein in the samples using a suitable bioassay, or immunoassay, or an equivalent assay. The data are typically displayed graphically as [glycoprotein] v. time and the bioavailability is expressed as the area under the curve (AUC). Relative bioavailability of a test preparation refers to the ratio between the AUC of the test preparation and that of the un-modified glycoprotein.

In some embodiments, the preparations of the present invention exhibit a relative bioavailability of at least about 110%, preferably at least about 120%, more preferably at least about 130% and most preferably at least about 140% of the bioavailability of the corresponding un-modified glycoprotein. The bioavailability may be measured in any mammalian species, preferably dogs, and the predetermined times used for calculating AUC may encompass different increments from 10 min- 8 h. Bioavailability may, for example, be measured in a dog model as follows: The experiment is performed as a four leg cross-over study in 12 Beagle dogs divided in four groups. All animals receive a test preparation A and a reference preparation B at a dose of about 90 µg/kg in a suitable buffer such as glycylglycine buffer (pH 5.5) containing sodium chloride (2.92 mg/ml), calcium chloride dihydrate (1.47 mg/ml), mannitol (30 mg/ml) and polysorbate 80. Blood samples are drawn at 10, 30, and 60 minutes and 2, 3, 4, 6 and 8 hours following the initial administration. Plasma is obtained from the samples and glycoprotein is quantified by ELISA.

The term "Immunogenicity" of a preparation refers to the ability of the preparation, when administered to a human, to elicit a deleterious immune response, whether humoral, cellular, or both. In any human sub-population, there may exist individuals who exhibit sensitivity to particular administered proteins. Immunogenicity may be measured by quantifying the presence of anti-glycoprotein antibodies and/or glycoprotein responsive T-cells in a sensitive individual, using conventional methods known in the art. In some embodiments, the modified glycoproteins of the present invention exhibit a decrease in immunogenicity in a sensitive individual of at least about 10%, preferably at least about 25%, more preferably at least about 40% and most preferably at least about 50%, relative to the immunogenicity for that individual of the un-modified glycoprotein.

Immunogenicity of a drug also relates to the fact that proteinaceous drugs may be immunogenic in non-sensitive subjects, meaning that repeated administrations of the drug leads to continuous boosting of an immune response against the drug. This is in most cases undesirable because the immune response will interfere with the activity of the drug, whereby it becomes necessary to administer increasing dosages of the drug over time in order to provide a therapeutic effect. In some embodiments, the modified glycoproteins of the present invention exhibit a decrease in immunogenicity in non-sensitive subjects of at least about 10%, preferably at least about 25%, more preferably at least about 40% and most preferably at least about 50%, relative to the immunogenicity for that individual of the un-modified glycoprotein.

The term "protease protected" as used herein referring to a glycoprotein means a glycoprotein which has been chemically modified in order to render said compound resistant to the plasma peptidases or proteases. Proteases in plasma are known to be involved in the degradation of several peptide hormones and also play a role in degradation of larger proteins.

Resistance of a glycoprotein to degradation by for instance dipeptidyl aminopeptidase IV (DPPIV) is determined by the following degradation assay: Aliquots of the glycoprotein (5 nmol) are incubated at 37 °C with 1 µL of purified dipeptidyl aminopeptidase IV corresponding to an enzymatic activity of 5 mU for 10-180 minutes in 100 µL of 0.1 M triethylamine-HCl buffer, pH 7.4. Enzymatic reactions are terminated by the addition of 5 µL of 10% trifluoroacetic acid, and the peptide degradation products are separated and quantified using HPLC analysis. One method for performing this analysis is : The mixtures are applied onto a Vydac C18 widepore (30 nm pores, 5 µm particles) 250 x 4.6 mm column and eluted at a flow rate of 1 ml/min with linear stepwise gradients of acetonitrile in 0.1% trifluoroacetic acid (0% acetonitrile for 3 min, 0-24% acetonitrile for 17 min, 24-48% acetonitrile for 1 min) according to Siegel et al., Regul. Pept. 1999;79:93-102 and Mentlein et al. Eur. J. Biochem. 1993;214:829-35. Peptides and their degradation products may be monitored by their absorbance at 220 nm (peptide bonds) or 280 nm (aromatic amino acids), and are quantified by integration of their peak areas related to those of standards. The rate of hydrolysis of a peptide by dipeptidyl aminopeptidase IV is estimated at incubation times which result in less than 10% of the peptide being hydrolysed.

The most abundant protein component in circulating blood of mammalian species is serum albumin, which is normally present at a concentration of approximately 3 to 4.5 grams per 100 milliters of whole blood. Serum albumin is a blood protein of approximately 70,000 daltons which provides several important functions in the circulatory system. For instance, it functions as a transporter of a variety of organic molecules found in the blood, as the main transporter of various metabolites such as fatty acids and bilirubin through the blood, and, owing to its abundance, as an osmotic regulator of the circulating blood. Serum albumin has a half-life of more than one week, and one approach to increasing the plasma half-life of peptides has been to derivatize the peptides with a chemical entity that binds to serum albumin. The term "albumin binder" refers to such chemical entities that are known to bind to plasma proteins, such as albumin. Albumin binding property may be determined as described in J.Med.Chem, 43, 2000, 1986-1992. Albumin binding moieties may include fatty acid derivatives, organic sulfatated polyaromates such as cibacron, as well as peptides comprising less than 40 amino acid residues such as moieties disclosed in J. Biol Chem. 277, 38 (2002) 35035-35043.

The modified glycoproteins, prepared according to the present invention exhibit improved functional properties relative to the un-modified glycoprotein. The improved functional properties may include, without limitation, a) physical properties such as, e.g., improved storage stability; b) improved pharmacokinetic properties such as, e.g., increased bioavailability and half-life; and c) reduced immunogenicity in humans.
Storage stability of a glycoprotein may be assessed by measuring (a) the time required for 20% of the bioactivity of a preparation to decay when stored as a dry powder at 25°C and/or (b) the time required for a doubling in the proportion of predetermined degradation products, such as, e.g., aggregates, in the preparation.

In some embodiments, the modified glycoproteins of the invention exhibit an increase of at least about 30%, preferably at least about 60% and more preferably at least about 100%, in the time required for 20% of the bioactivity to decay relative to the time required for the un-modified glycoprotein, when a preparation comprising the glycoprotein are stored as dry powders at 25°C.

Bioactivity measurements may be performed in accordance with the kind of bioactivity associated with the particular protein; in case of, e.g., coagulation factors, bioactivity may be measured using any of a clotting assay, proteolysis assay, TF-binding assay, or TF-independent thrombin generation assay.

In some embodiments, the preparations of the invention exhibit an increase of at least about 30%, preferably at least about 60%, and more preferably at least about 100%, in the time required for doubling of predetermined degradation products, such as, e.g., aggregates, relative to a reference preparation, when both preparations are stored as dry powders at 25°C. The content of aggregates may, for example, be determined by gel permeation HPLC, or another type of well-known chromatography methods. In the case of coagulation factors, aggregates may be determined by gel permeation HPLC on a Protein Pak 300 SW column (7.5 x 300 mm) (Waters, 80013) as follows. The column is equilibrated with Eluent A (0.2 M ammonium sulfate, 5 % isopropanol, pH adjusted to 2.5 with phosphoric acid, and thereafter pH is adjusted to 7.0 with triethylamine), after which 25 µg of sample is applied to the column. Elution is with Eluent A at a flow rate of 0.5 ml/min for 30 min, and detection is achieved by measuring absorbance at 215 nm. The content of aggregates is calculated as the peak area of the coagulation factors aggregates/total area of coagulation factor peaks (monomer and aggregates).

### The bivalent linker L and L':

L and L' represents a bivalent chemical linker, which may be identical or may be different.

In one embodiment L and L' independently represents a bond or

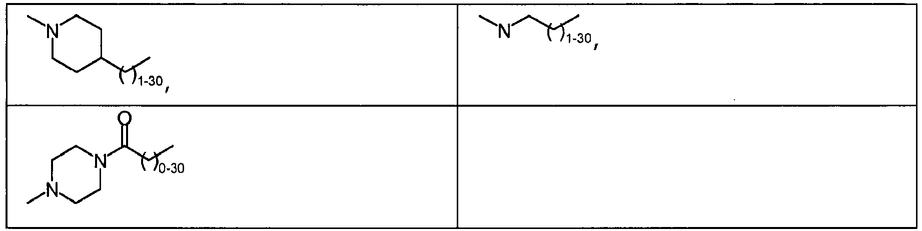

### The substituents_M and M'

In the following, the substituent M and/or M' will be discussed.
In one embodiment of the invention, increased half-life is obtained by M and/or M' being a group that increases molecular weight so that renal clearance is reduced or abolished and/or by M and/or M' being a group that masks binding partners for hepatic receptors. In an alternative embodiment, the reduced immunogenicity is obtained by M and/or M' being a group which blocks antibody binding to immunogenic sites. In yet another embodiment, improved affinity for albumin is obtained by M and/or M' being a group which has high affinity for albumin. And in yet another embodiment improved affinity for a receptor is obtained by M and/or M' being a group which specifically binds a surface receptor on a target cell.
The substituent M and/or M' can be any functionality improving group, e.g. a "protractor group". As used herein this means a group which upon conjugation to a protein or peptide increases the circulation half-life of said protein or peptide, when compared to the unmodified protein or peptide. The specific principle behind the protractive effect may be caused by increased size, shielding of peptide sequences that can be recognized by peptidases or antibodies, or masking of glycanes in such way that they are not recognized by glycan specific receptores present in e.g. the liver or on macrophages, preventing or decreasing clearance. The protractive effect of the protractor group can e.g. also be caused by binding to blood components such as albumin, or by specific or unspecific adhesion to vascular tissue. The conjugated glycoprotein should substantially preserve biological activity of the non-modified glycoprotein.

Other possibilities include those where M and/or M' is a group that targets the modified glycoprotein to a certain type of cell or tissue, as is e.g. of interest if the glycoprotein has to exert its effect at a very high local concentration. Yet further possibilities include those where M and/or M' is in its own right an active principle, e.g. a radionuclide or a toxic substance - this can e.g. be convenient in cases where the unmodified glycoprotein has high affinity for a receptor in malignant tissue and thus functions as a targeting moiety in the modified molecule.

In one embodiment of the invention M and/or M' is selected from the group consisting of:
- A low molecular organic charged radical (15-1000 Da), which may contain one or more carboxylic acids, amines sulfonic acids, phosphonic acids, or combination thereof,
- A low molecular (15-1000 Da) neutral hydrophilic molecule, such as cyclodextrin, or a polyethylene chain which may optionally branched,
- A low molecular (15-1000 Da) hydrophobic molecule such as a fatty acid or cholic acid or derivatives theroff,
- Polyethyleneglycol with an avarage molecular weight of 2-40 KDa,
- A well defined precission polymer such as a dendrimer with an exact molecular mass ranging from 700 to 20.000 Da, or more preferably between 700-10.000 Da,
- A substantially non imunogenic glycoprotein such as albumin or an antibody or part of an antibody optionally containing a Fc-domain, and
- A high molecular weight organic polymer such as dextran.

In one embodiment of the invention the polymeric molecule is selected from the group consisting of dendrimers (e.g. with a molecular weight in the range of 700-10.000 Da or dendrimers as disclosed in International Patent Application WO 2005014049), polyalkylene oxide (PAO), including polyalkylene glycol (PAG), such as polyethylene glycol (PEG) and polypropylene glycol (PPG), branched PEGs, polyvinyl alcohol (PVA), polycarboxylate, poly-vinylpyrolidone, polyethylene-co-maleic acid anhydride, polystyrene-co-maleic acid anhydride, and dextran, including carboxymethyl-dextran, HES, PHF or MPC. In one embodiment of the invention, the polymeric molecule is a PEG group. In one embodiment of the invention, the polymeric molecule is a dendrimer.

In one embodiment of the invention, M and/or M' is a protractor group selected from the group consisting of serum protein binding-ligands, such as serum protein binding-ligands, such as compounds which bind to albumin, such as fatty acids, C5-C24 fatty acid, aliphatic diacid (e.g. C5-C24), a structure (e.g. sialic acid derivatives or mimetics) which inhibits the glycans from binding to receptors (e.g. asialoglycoprotein receptor and mannose receptor), a small organic molecule containing moieties that under physiological conditions alters charge properties, such as carboxylic acids or amines, or neutral substituents that prevent glycan specific recognition such as smaller alkyl substituents (e.g., C1-C5 alkyl), a low molecular organic charged radical (e.g. C1-C25), which may contain one or more carboxylic acids, amines, sulfonic, phosphonic acids, or combination thereof; a low molecular neutral hydrophilic molecule (e.g. C1-C25), such as cyclodextrin, or a polyethylene chain which may optionally branched; polyethyleneglycol with an avarage molecular weight of 2-40 KDa; a well defined precission polymer such as a dendrimer with an exact molecular mass ranging from 700 to 20.000 Da, or more preferably between 700-10.000 Da; and a substantially non-imunogenic glycoprotein such as albumin or an antibody or part of an antibody optionally containing a Fc-domain.

In one embodiment M and/or M' are selected independently from:

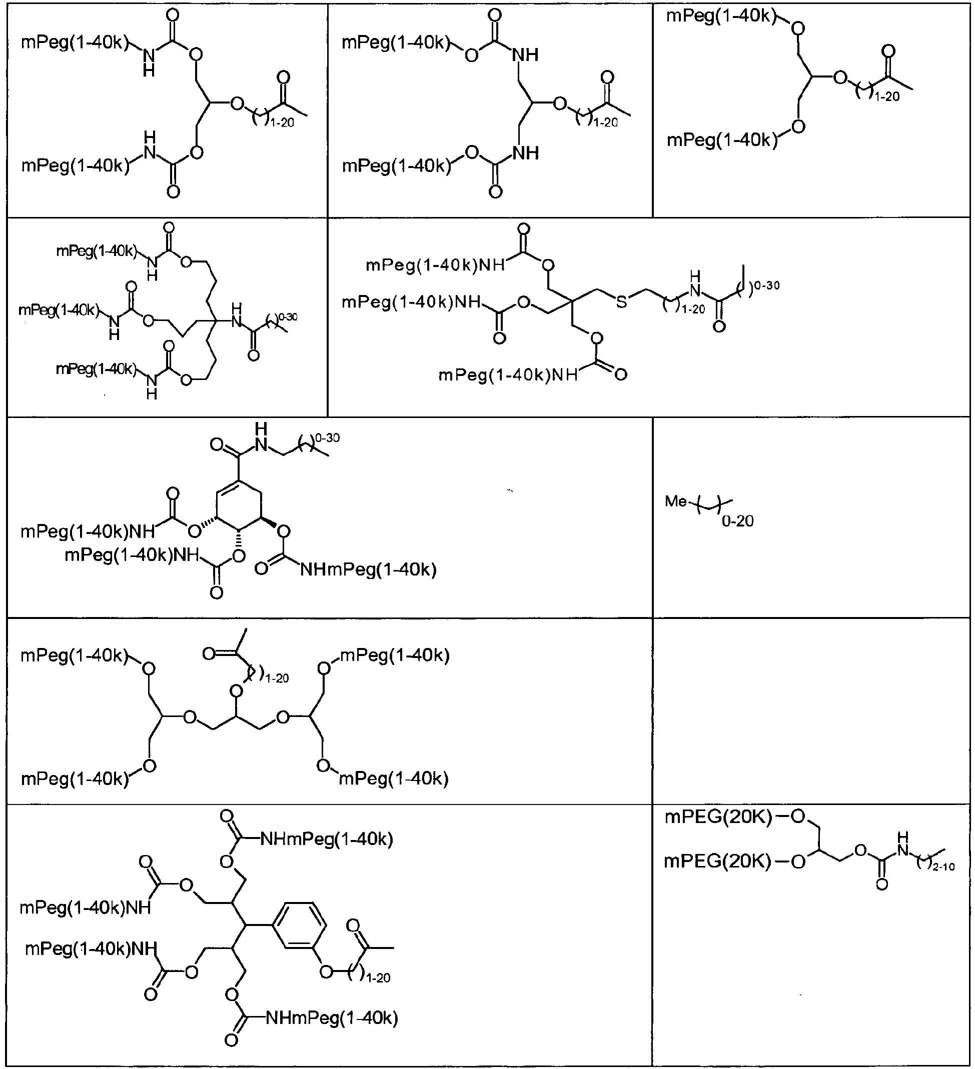

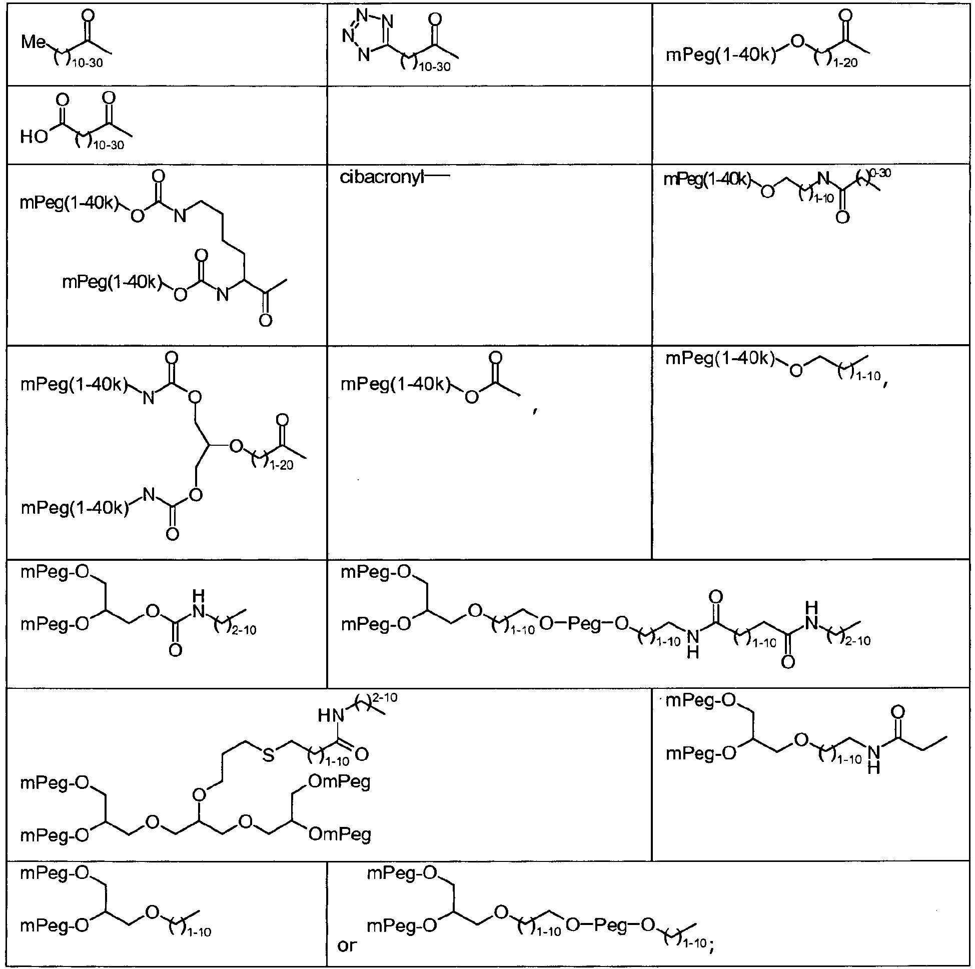

In one embodiment M and/or M' are selected independently from:

| | | |
|---|---|---|
| 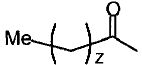 | 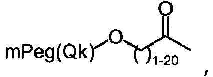 | 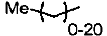 |
| wherein Z is 14, 16, 18 or 20, | | |
| 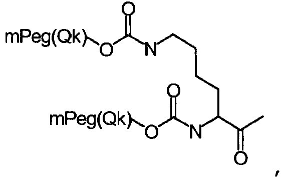 | 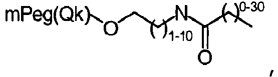 | |
| 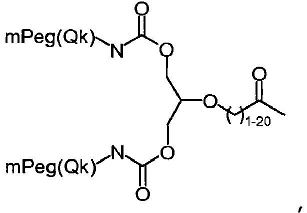 | 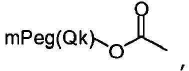 | 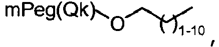 |
| 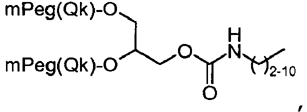 | | |
| 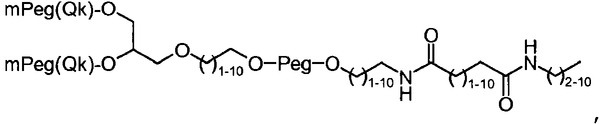 | | |
| 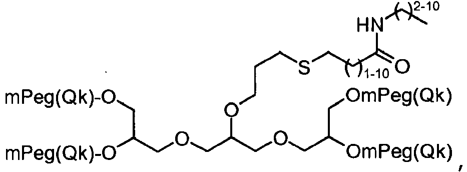 | | 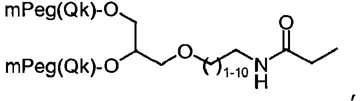 |
| 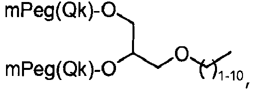 | 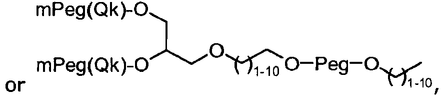 | |

wherein Q represents an integer from 10-20, 10-30, 10-40, 20-30, 20-40, 30-40, such as 10, 20 or 30. 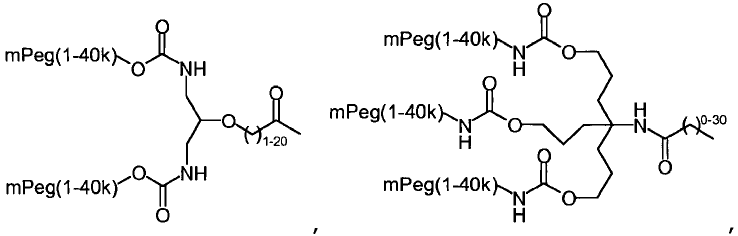 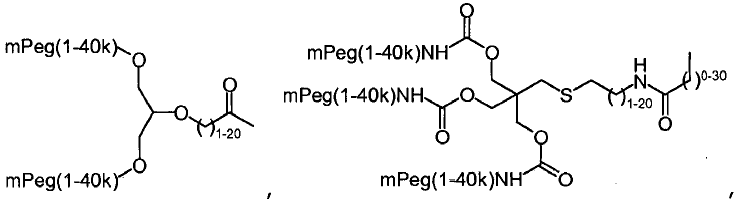 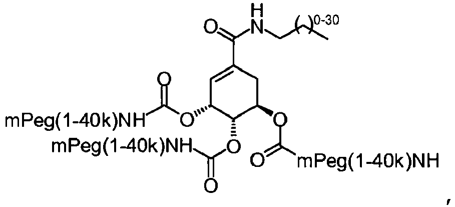 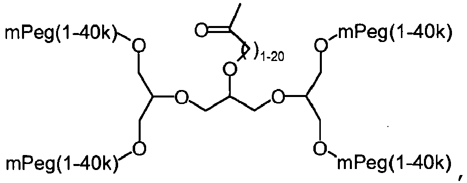 or

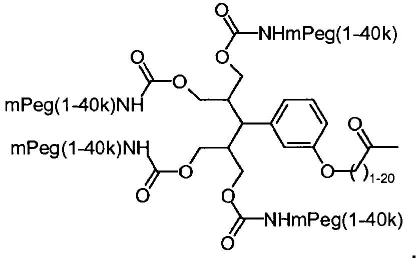

In one embodiment, M-L-ONH2 and/or M'L'-ONH2 is: 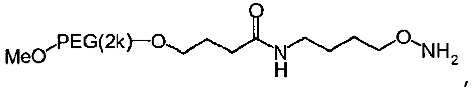 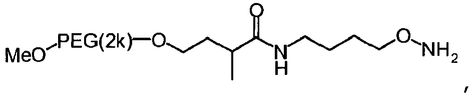 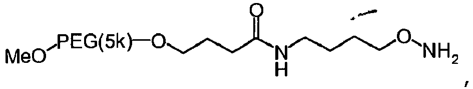 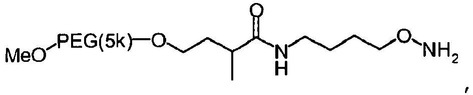 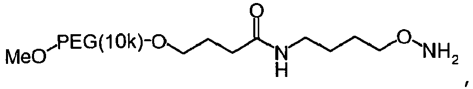 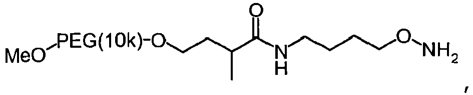 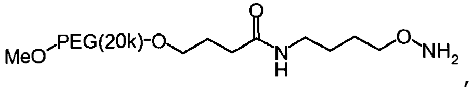 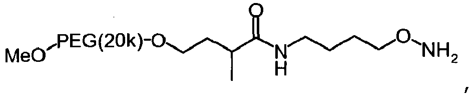 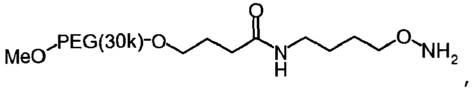 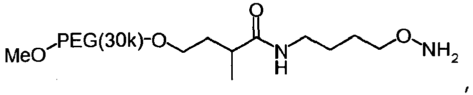 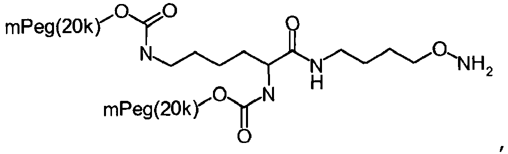 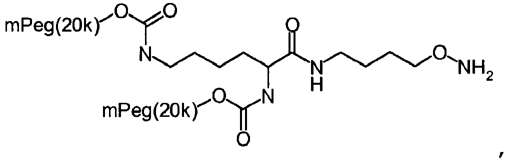 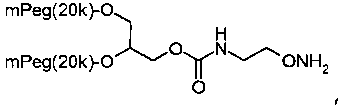 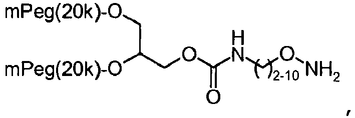 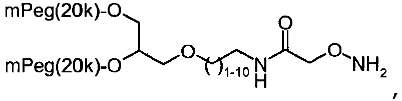 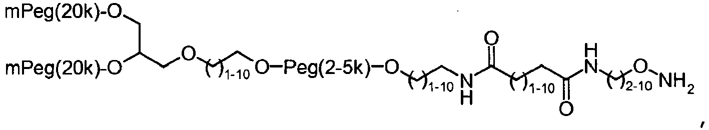 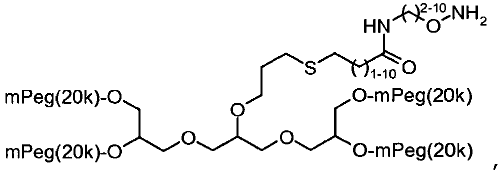 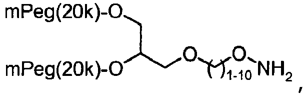 and 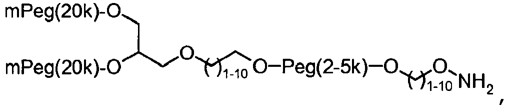 and

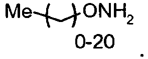

In one embodiment M-L-ONH2 and/or M'-L'-ONH2 are indepentently selected amongst: 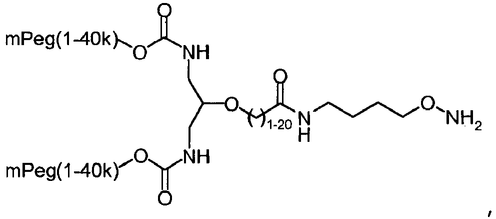 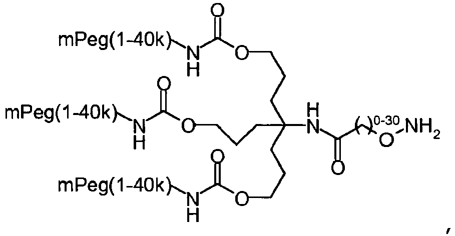 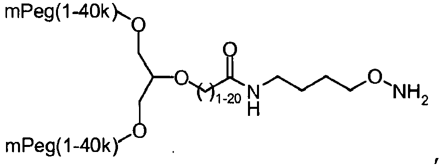 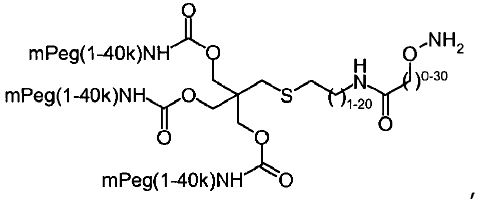 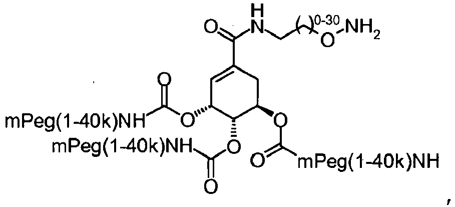 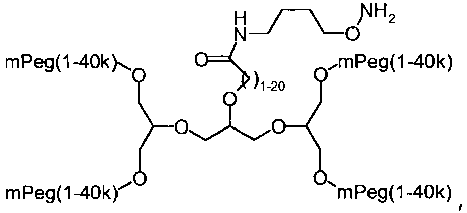 and

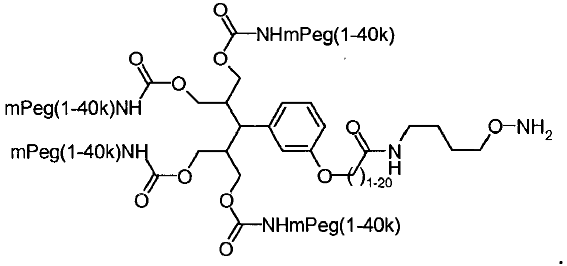

In one embodiment, M-L-ONH2 and/or M'-L'-ONH2 is independently selected amongst 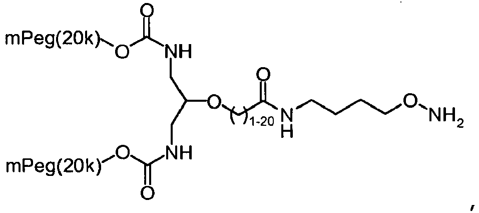 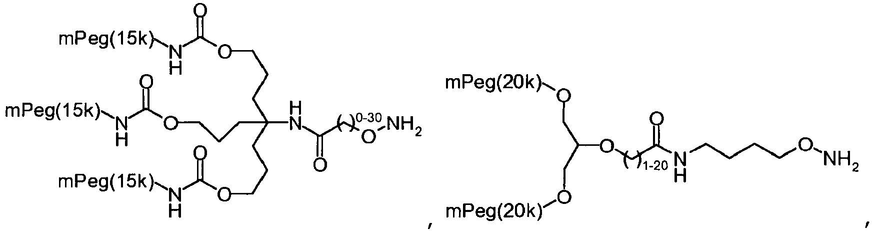 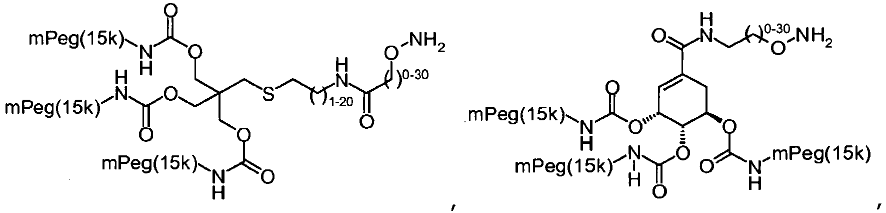 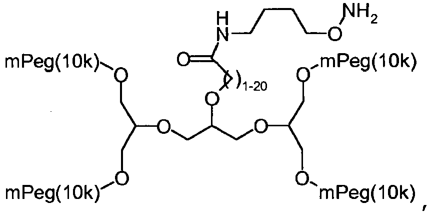 and

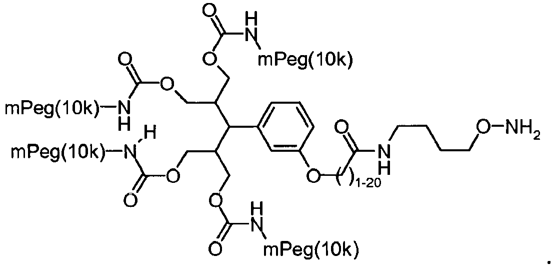

In one embodiment M-L-ONH2 and/or M'-L'-ONH2 is independently selected amongst 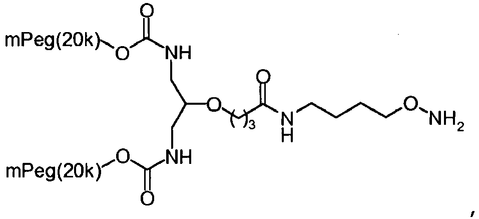 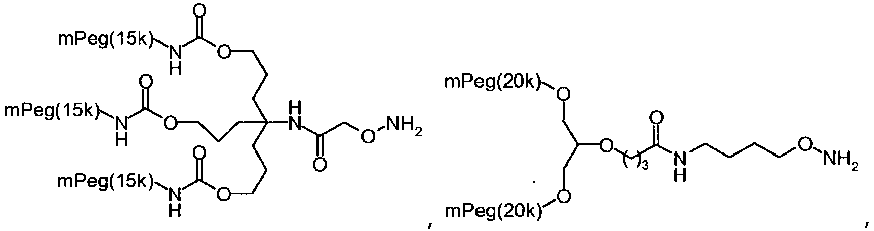 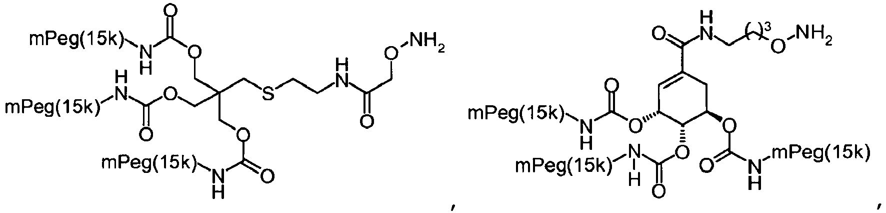 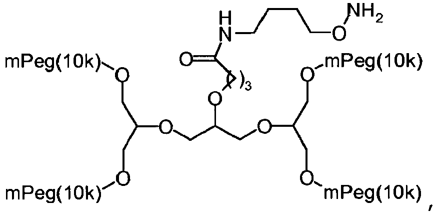 and 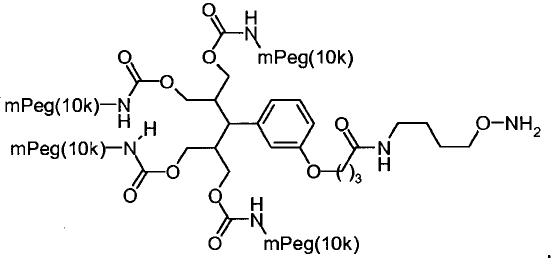 M and/or M' may be an organic radical selected from one of the groups below:
- straight, branched and/or cyclic C₁₋₃₀alkyl, C₂₋₃₀alkenyl, C₂₋₃₀alkynyl, C₁₋₃₀heteroalkyl, C₂₋₃₀heteroalkenyl, C₂₋₃₀heteroalkynyl, wherein one or more homocyclic aromatic compound biradical or heterocyclic compound biradical may be inserted, and wherein said C₁₋₃₀ or C₂₋₃₀ radicals may optionally be substituted with one or more substituents selected from -CO₂H, -SO₃H, -PO₂OH, -SO₂NH₂, -NH₂, -OH, -SH, halogen, or aryl, wherein said aryl is optionally substituted with -CO₂H, -SO₃H, PO₂OH, -SO₂NH₂, -NH₂, -OH, -SH, or halogen; steroid radicals; lipid radicals;
- polysaccharide radicals, e.g. dextrans; α-, β-, or γ-cyclodextrin, polyamide radicals e.g. polyamino acid radicals; PVP radicals; PVA radicals; poly(1-3-dioxalane); poly(1,3,6-trioxane); ethylene/maleic anhydride polymer;
- Cibacron dye stuffs, such as Cibacron Blue 3GA, and polyamide chains of specified length, as disclosed in WO 00/12587;
- a substantially non-immunogenic protein residue such as a blood component like albuminyl derivative, or an antibody or a domain thereof such as a Fc domain from human normal IgG1, as described in Kan, SK et al in The Journal of Immunology 2001, 166(2), 1320-1326 or in Stevenson, GT, The Journal of Immunology 1997, 158, 2242-2250;
- polyethylene glycol (PEG) or methoxy polyethylene glycol (mPEG) radicals and amino derivatives thereof, where the avarage molecular weight may be between 500 and 100,000 Da, such as between 500 and 60,000 Da, such as between 1000 and 40,000 Da, such as between 5000 and 40,000 Da;
- moieties that are known to bind to plasma proteins, such as e.g. albumin, where the albumin binding property may be determined as described in J.Med.Chem, 43, 2000, 1986-1992, or an albumin binding moiety such as a peptide comprising less than 40 amino acid residues such as moieties disclosed in J. Biol Chem. 277, 38 (2002) 35035-35043.

In other embodiments, M and/or M' is C₁-C₂₀-alkyl, such as C₁-C₁₈-alkyl. Specific mentioning is made of C₁₄-, C₁₆- and C₁₈-alkyl, which optionally may be substituted with in particular charged groups, polar groups and/or halogens. Examples of such substituents include -CO₂H, tetrazol and halogen. In a particular embodiment, all hydrogens in the C₁-C₂₀-alkyl are substituted with fluoro to form perfluoroalkyl.

In particular embodiments M' is different from M. In particular embodiments M' has a substantially lower molecular weight than M. In one particular embodiment, M' is methoxyl amin (MeONH₂)

### The starting molecule P*

In the following, the substituent P* will be discussed.

The Glycoprotein P* comprises a glycan terminal, that is reactive to oxidation by periodate.

Thus, the reactive group in P* is part of a carbohydrate, or derived from a carbohydrate residue such as those found in N- or O-glycanes of glycosylated glycoproteins. Alternatively the reactive group of a glycoprotein P* may be a sialic acid residue.

In one embodiment P* is selected from FVII, FVIII, FIX, FX, FII, FV, protein C, protein S, tPA, PAI-1, tissue factor, FXI, FXII, FXIII, as well as sequence variants thereof; immunoglobulins, cytokines such as interleukins, alpha-, beta-, and gamma-interferons, colony stimulating factors including granulocyte colony stimulating factors, fibroblast growth factors, platelet derived growth factors and phospholipase-activating protein (PUP). P* can also be any other protein and peptide of general biological and therapeutic interest include insulin, plant proteins such as lectins and ricins, tumor necrosis factors and related alleles, soluble forms of tumor necrosis factor receptors, interleukin receptors and soluble forms of interleukin receptors, growth factors such as tissue growth factors, such as TGFa's or TGFps and epidermal growth factors, hormones, somatomedins, erythropoietin, pigmentary hormones, hypothalamic releasing factors, antidiuretic hormones, prolactin, chorionic gonadotropin, follicle-stimulating hormone, thyroid-stimulating hormone, tissue plasminogen activator, and immunoglobulins such as IgG, IgE, IgM, IgA, and IgD, and fragments thereof.

Peptides and proteins, that do not contain glycan terminals can be glycosylated either enzymatically as described in Li Shao et all. Glycobiology 12(11) 762-770 (2002) using glycosyltransferases, or chemically synthesised , for example by using standard peptide chemistry and glycosylated amino acid components such as N-galactosylated asparagine.

Alternatively glycosylation sites may be engineered into proteins or peptides which in vivo normally are produced in their non-glycosylated form. For example insertion of the consensus sequence Cys-XXX-Ser-XXX-Pro-Cys in an EGF repeat allows for selective O-glycosylation of serine using UDP-Glucose and glucosyltransferase (Li Shao et all. Glycobiology 12(11) 762-770 (2002)), whereas insertion of the consensus sequence Asn-XXX-Ser/Thr allows for N-glycosylation (R.A. Dwek, Chem. Rev. 1996, 96, 683-720). Peptide sequences containing threonine or serine also undergoes glycosylation in the presence of UDP-GalNAc:polypeptide N-acetylgalactosaminyltransferase and UDP-GalNAc in a sequence dependent manner (see for example B.C. O'Connell, F.K.Hagen and L.A. Tabak in J. Biol. Chem. 267(35), 25010-25018 (1992)). Alternatively site directed mutagenesis introducing cystein mutations can be used for introduction of galactose or galactose containing sugar structures via mixed disulphide fromation as described by D.P. Gamblin et al. in Angew. Chem.Int. Ed., 43, 828 (2004). Galactose or N-acetylgalactosamine containing peptide and proteins can also be made by conjugation to proteins or peptides containing non-biogenical handles such as methods described by P.G. Schultz in J.Am.Chem.Soc, 125, 1702 (2003), or unspecifically by direct glycosylation of peptides using glycosyl donor substrates such as trichloroacetamidyl galactosides ect. Addition of glycosidase inhibitores to fermentation cultures, thereby producing glycoproteins with truncated glycan structures as described in US 4925796A / US 5272066A1 is also a possibility for obtaining galactose or N-acetylgalactosamine containing proteins, as well as enzymatic modification of glutamine residues using TGase (see for example M. Sato et al. Angew. Chem. Int. Ed. 43, 1516-1520, (2004)).

Production og N-glycosylated proteins are not limited to the use of mammalian host cells such as CHO or BHK cells, but also can be performed in insect cells, yeast, or by using bacterial cells as described by M. Wacker et al. in Science, 298, 1790-1793 (2002). In an embodiment of the invention the peptide is aprotinin, tissue factor pathway inhibitor or other protease inhibitors, insulin or insulin precursors, human or bovine growth hormone, interleukin, glucagon, oxyntomodulin,GLP-1, GLP-2, IGF-I, IGF-II, tissue plasminogen activator, transforming growth factor γ or β, platelet-derived growth factor, GRF (growth hormone releasing factor), human growth factor, immunoglobulines, EPO, TPA, protein C, blood coagulation factors such as FVII, FVIII, FIX, FX, FII, FV, protein C, protein S, PAI-1, tissue factor, FXI, FXII, and FXIII, exendin-3, exentidin-4, and enzymes or functional analogues thereof. In the present context, the term "functional analogue" is meant to indicate a protein with a similar function as the native protein. The protein may be structurally similar to the native protein and may be derived from the native protein by addition of one or more amino acids to either or both the C and N-terminal end of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Furthermore the protein may be acylated in one or more positions, see, e.g., WO 98/08871, which discloses acylation of GLP-1 and analogues thereof, and WO 98/08872, which discloses acylation of GLP-2 and analogues thereof. An example of an acylated GLP-1 derivative is Lys26(N^{epsilon}-tetradecanoyl)-GLP-1 (7-37) which is GLP-1 (7-37) wherein the epsilon-amino group of the Lys residue in position 26 has been tetradecanoylated.

The proteins or portions thereof can be prepared or isolated by using techniques known to those of ordinary skill in the art such as tissue culture, extraction from animal sources, or by recombinant DNA methodologies. Transgenic sources of the proteins, peptides, amino acid sequences and the like are also contemplated. Such materials are obtained form transgenic animals. i. e., mice, pigs, cows, etc., wherein the proteins expressed in milk, blood or tissues. Transgenic insects and baculovirus expression systems are also contemplated as sources. Moreover, mutant versions, of proteins, such as mutant TNF's and/or mutant interferons are also within the scope of the invention. Other proteins of interest are allergen proteins such as ragweed, Antigen E, honeybee venom, mite allergen, and the like.

The foregoing is illustrative of the biologically active peptides which are suitable for conjugation with a protractor group in accordance with the invention. It is to be understood that those biologically active materials not specifically mentioned but having suitable peptides are also intended and are within the scope of the present invention.
In one embodiment, the glycoprotein is a FVII polypeptide. In one embodiment, the polypeptides are wild-type Factor VIIa.

As used herein, the terms "Factor VII polypeptide " or "FVII polypeptide" means any protein comprising the amino acid sequence 1-406 of wild-type human Factor VIIa (i.e., a polypeptide having the amino acid sequence disclosed in U.S. Patent No. 4,784,950), as well as variants thereof.

The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like.

As used herein, "wild type human FVIIa" is a polypeptide having the amino acid sequence disclosed in U.S. Patent No. 4,784,950.

Non-limiting examples of Factor VII variants include S52A-FVIIa, S60A-FVIIa ( Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189 (corresponding to WO 02/077218); and FVII variants exhibiting increased proteolytic stability as disclosed in WO 02/38162 (Scripps Research Institute); FVII variants having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and FVII variants as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of FVII variants having increased biological activity compared to wild-type FVIIa include FVII variants as disclosed in WO 01/83725, WO 02/22776, WO 02/077218, PCT/DK02/00635 (corresponding to WO 03/027147), Danish patent application PA 2002 01423 (corresponding to WO 04/029090), Danish patent application PA 2001 01627 (corresponding to WO 03/027147); WO 02/38162 (Scripps Research Institute); and FVIIa variants with enhanced activity as disclosed in JP 2001061479 (Chemo-Sero-Therapeutic Res Inst.).

Examples of variants of factor VII include, without limitation, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V158T/E296V/M298Q-FVII, S314E/L305V/V158T/K337A/M298Q-FVII, S314E/L305V/V158T/E296V/K337A-FVII, S314E/L305V/V158D/K337A/M298Q-FVII, S314E/L305V/V158D/E296V/K337A-FVII, S314E/L305V/V158D/E296V/M298Q/K337A-FVII, S314E/L305V/V158T/E296V/M298Q/K337A-FVII, K316H/L305V/K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305V/V158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A -FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A -FVII, K316Q/L305V/V158D/E296V/M298Q/K337A-FVII, K316Q/L305V/V158T/E296V/M298Q/K337A-FVII, F374Y/K337A-FVII, F374Y/V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/L305V/V158D-FVII, F374Y/L305V/E296V-FVII, F374Y/L305V/M298Q-FVII, F374Y/L305V/V158T-FVII, F374Y/L305V/S314E-FVII, F374Y/K337A/S314E-FVII, F374Y/K337A/V158T-FVII, F374Y/K337A/M298Q-FVII, F374Y/K337A/E296V-FVII, F374Y/K337A/V158D-FVII, F374Y/V158D/S314E-FVII, F374Y/V158D/M298Q-FVII, F374Y/V158D/E296V-FVII, F374Y/V158T/S314E-FVII, F374Y/V158T/M298Q-FVII, F374Y/V158T/E296V-FVII, F374Y/E296V/S314E-FVII, F374Y/S314E/M298Q-FVII, F374Y/E296V/M298Q-FVII, F374Y/L305V/K337A/V158D-FVII, F374Y/L305V/K337A/E296V-FVII, F374Y/L305V/K337A/M298Q-FVII, F374Y/L305V/K337A/V158T-FVII, F374Y/L305V/K337A/S314E-FVII, F374Y/L305V/V158D/E296V-FVII, F374Y/L305V/V158D/M298Q-FVII, F374Y/L305V/V158D/S314E-FVII, F374Y/L305V/E296V/M298Q-FVII, F374Y/L305V/E296V/V158T-FVII, F374Y/L305V/E296V/S314E-FVII, F374Y/L305V/M298Q/V158T-FVII, F374Y/L305V/M298Q/S314E-FVII, F374Y/L305V/V158T/S314E-FVII, F374Y/K337A/S314E/V158T-FVII, F374Y/K337A/S314E/M298Q-FVII, F374Y/K337A/S314E/E296V-FVII, F374Y/K337A/S314E/V158D-FVII, F374Y/K337A/V158T/M298Q-FVII, F374Y/K337A/V158T/E296V-FVII, F374Y/K337A/M298Q/E296V-FVII, F374Y/K337A/M298Q/V158D-FVII, F374Y/K337A/E296V/V158D-FVII, F374Y/V158D/S314E/M298Q-FVII, F374Y/V158D/S314E/E296V-FVII, F374Y/V158D/M298Q/E296V-FVII, F374Y/V158T/S314E/E296V-FVII, F374Y/V158T/S314E/M298Q-FVII, F374Y/V158T/M298Q/E296V-FVII, F374Y/E296V/S314E/M298Q-FVII, F374Y/L305V/M298Q/K337A/S314E-FVII, F374Y/L305V/E296V/K337A/S314E-FVII, F374Y/E296V/M298Q/K337A/S314E-FVII, F374Y/L305V/E296V/M298Q/K337A -FVII, F374Y/L305V/E296V/M298Q/S314E-FVII, F374Y/V158D/E296V/M298Q/K337A-FVII, F374Y/V158D/E296V/M298Q/S314E-FVII, F374Y/L305V/V158D/K337A/S314E-FVII, F374Y/V158D/M298Q/K337A/S314E-FVII, F374Y/V158D/E296V/K337A/S314E-FVII, F374Y/L305V/V158D/E296V/M298Q-FVII, F374Y/L305V/V158D/M298Q/K337A-FVII, F374Y/L305V/V158D/E296V/K337A-FVII, F374Y/L305V/V158D/M298Q/S314E-FVII, F374Y/L305V/V158D/E296V/S314E-FVII, F374Y/V158T/E296V/M298Q/K337A-FVII, F374Y/V158T/E296V/M298Q/S314E-FVII, F374Y/L305V/V158T/K337A/S314E-FVII, F374Y/V158T/M298Q/K337A/S314E-FVII, F374Y/V158T/E296V/K337A/S314E-FVII, F374Y/L305V/V158T/E296V/M298Q-FVII, F374Y/L305V/V158T/M298Q/K337A-FVII, F374Y/L305V/V158T/E296V/K337A-FVII, F374Y/L305V/V158T/M298Q/S314E-FVII, F374Y/L305V/V158T/E296V/S314E-FVII, F374Y/E296V/M298Q/K337A/V158T/S314E-FVII, F374Y/V158D/E296V/M298Q/K337A/S314E-FVII, F374Y/L305V/V158D/E296V/M298Q/S314E-FVII, F374Y/L305V/E296V/M298Q/V158T/S314E-FVII, F374Y/L305V/E296V/M298Q/K337A/V158T-FVII, F374Y/L305V/E296V/K337A/V158T/S314E-FVII, F374Y/L305V/M298Q/K337A/V158T/S314E-FVII, F374Y/L305V/V158D/E296V/M298Q/K337A-FVII, F374Y/L305V/V158D/E296V/K337A/S314E-FVII, F374Y/L305V/V158D/M298Q/K337A/S314E-FVII, F374Y/L305V/E296V/M298Q/K337A/V158T/S314E-FVII, F374Y/L305V/V158D/E296V/M298Q/K337A/S314E-FVII, S52A-Factor VII, S60A-Factor VII; R152E-Factor VII, S344A-Factor VII, T106N-FVII, K143N/N145T-FVII, V253N-FVII, R290N/A292T-FVII, G291N-FVII, R315N/V317T-FVII, K143N/N145T/R315N/V317T-FVII; and FVII having substitutions, additions or deletions in the amino acid sequence from 233Thr to 240Asn; FVII having substitutions, additions or deletions in the amino acid sequence from 304Arg to 329Cys; and FVII having substitutions, additions or deletions in the amino acid sequence from 153Ile to 223Arg.

In one embodiment, the glycoprotein is a FVIII polypeptide. In one embodiment, the glycoprotein is a FIX polypeptide.

### Other aspects of the invention

The present invention also pertains to novel modified glycoproteins with the general structure

(M-L-O-N=CH)ₙ-P-(CH=N-O-L'-M')ₘ (formula I)

wherein M and optionally M' independently is a polymeric moiety for increasing the molecular weight of the modified glycoprotein, and wherein L and L' independently represent a bivalent linker, and wherein P represents a glycoprotein comprising one or more oxidized glucan terminals of said glycoprotein, O, N, C and H represents oxygen, nitrogen, carbon and hydrogen atoms respectively, n is 1-10, m is 0-50, wherein said modified glycoprotein has improved pharmacologic properties compared to the starting glycoprotein P* and has retained its functional activity.

Such a modified glycoprotein is in some embodiments of the invention selected from modified FVII, FVIII, FIX, FX, FII, FV, protein C, protein S, tPA, PAI-1, tissue factor, FXI, FXII, FXIII, as well as sequence variants thereof; immunoglobulins, cytokines such as interleukins, alpha-, beta-, and gamma-interferons, colony stimulating factors including granulocyte colony stimulating factors, fibroblast growth factors, platelet derived growth factors and phospholipase-activating protein (PUP).

Other modified glycoproteins are modified proteins and peptides of general biological and therapeutic interest, e.g. including insulin, plant proteins such as lectins and ricins, tumor necrosis factors and related alleles, soluble forms of tumor necrosis factor receptors, interleukin receptors and soluble forms of interleukin receptors, growth factors such as tissue growth factors, such as TGFa's or TGFps and epidermal growth factors, hormones, somatomedins, erythropoietin, pigmentary hormones, hypothalamic releasing factors, antidiuretic hormones, prolactin, chorionic gonadotropin, follicle-stimulating hormone, thyroid-stimulating hormone, tissue plasminogen activator, and immunoglobulins such as IgG, IgE, IgM, IgA, and IgD, and fragments thereof.

In one embodiment of the invention the modified glycoprotein is N-glycosylated and/or O-glycosylated and/or contains sialic acid moieties.

In one embodiment of the invention the modified glycoprotein has improved pharmacologic property selected from the group consisting of increased bioavailability, increased functional *in vivo* half-life, increased *in vivo* plasma half-life, reduced immunogenicity, increased protease resistance, increased affinity for albumin, improved affinity for a receptor, increased storage stability, decreased functional *in vivo* half-life, decreased *in vivo* plasma half-life. In one embodiment the increased half-life is obtained by M being a group that increases molecular weight so that renal clearance is reduced or abolished and/or by M being a group that masks binding partners for hepatic receptors. In one embodiment reduced immunogenicity is obtained by M being a group which blocks antibody binding to immunogenic sites. In one embodiment improved affinity for albumin is obtained by M being a group which has high affinity for albumin. In one embodiment improved affinity for a receptor is obtained by M being a group which specifically binds a surface receptor on a target cell.

In one embodiment of the invention the modified glycoprotein is wherein M is selected from the group consisting of: a low molecular weight organic charged radical, which may contain one or more carboxylic acids, amines, sulfonic acids, phosphonic acids, or combinations thereof; a low molecular weight neutral hydrophilic molecule, such as cyclodextrin or a optionally branched polyethylene chain; a low molecular weight hydrophobic molecule such as a fatty acid or cholic acid or derivatives thereof; a polyethylene glycol with an average molecular weight of 2-40 kDa; a well-defined precision polymer such as a dendrimer with an exact molecular mass ranging from 700 Da to 20 kDa; a substantially non-immunogenic polypeptide such as albumin, an antibody or a part of an antibody optionally containing a Fc-domain; and a high molecular weight organic polymer.

In one embodiment of the invention the modified glycoprotein is wherein M is selected from the group consisting of a dendrimer, polyalkylene oxide (PAO), including polyalkylene glycol (PAG), such as polyethylene glycol (PEG) and polypropylene glycol (PPG), branched PEG, polyvinyl alcohol (PVA), polycarboxylate, poly-vinylpyrolidone, polyethylene-co-maleic acid anhydride, polystyrene-co-maleic acid anhydride, dextran, carboxymethyl-dextran, HES, MPC or PHF.

In one embodiment of the invention the modified glycoprotein is wherein M is selected from the group consisting of a serum protein binding-ligand and a small organic molecule containing moieties that under physiological conditions alters charge properties, a structure which inhibits glycans from binding to receptors, and a neutral substituent that prevent glycan specific recognition.

In one embodiment the modified glycoprotein according to the invention is wherein P is selected from FVII, FVIII, FIX, FX, FII, FV, protein C, protein S, tPA, PAI-1, tissue factor, FXI, FXII, FXIII, as well as sequence variants thereof; immunoglobulins, cytokines such as interleukins, alpha-, beta-, and gamma-interferons, colony stimulating factors including granulocyte colony stimulating factors, fibroblast growth factors, platelet derived growth factors, phospholipase-activating protein (PUP), insulin, plant proteins such as lectins and ricins, tumor necrosis factors and related alleles, soluble forms of tumor necrosis factor receptors, interleukin receptors and soluble forms of interleukin receptors, growth factors such as tissue growth factors, such as TGFa's or TGFps and epidermal growth factors, hormones, somatomedins, erythropoietin, pigmentary hormones, hypothalamic releasing factors, antidiuretic hormones, prolactin, chorionic gonadotropin, follicle-stimulating hormone, thyroid-stimulating hormone, tissue plasminogen activator, and immunoglobulins such as IgG, IgE, IgM, IgA, and IgD, and fragments thereof, or any fusion proteins comprising any of the above mentioned proteins or fragments thereof.

In one embodiment the modified glycoprotein according to the invention is wherein the glycoprotein is a Factor VII polypeptide.

In one embodiment the modified glycoprotein according to the invention is wherein the glycoprotein has the amino acid sequence of wild-type human Factor VII.

In one-embodiment the modified glycoprotein according to the invention is wherein the modified glycoprotein exhibit at least about 10 %, such as at least about 20 %, such as at least about 40 %, such as at least about 60 %, such as at least about 80 %, such as at least about 100 % of the specific activity of un-modified Factor VII polypeptide when tested in one or more of a clotting assay, proteolysis assay, or TF binding assay as described in the present specification.

In one embodiment the modified glycoprotein according to the invention is wherein the modified glycoprotein exhibits a bioavailability that is at least about 110% of the bioavailability of the un-modified glycoprotein, such as at least about 120%, about 130%, or at least about 140% of the bioavailability of the un-modified glycoprotein.

In one embodiment the modified glycoprotein according to the invention is wherein the modified glycoprotein exhibits a serum half-life that is at least about 125% of the half-life of the un-modified glycoprotein, such as about 150%, about 200%, or at least about 250% of the half-life of the un-modified glycoprotein.

In one embodiment, the method for production of the modified glycosylated molecules comprises the further step of formulating said glycosylated molecule as a pharmaceutical composition.

In one embodiment of the invention, m in the modified glycoproteins with the general structure (M-L-O-N=CH)ₙ-P-(CH=N-O-L'-M')ₘ is in the range of 0-50, such as 0-20, such as 0-10, such as 0-5, such as 0-3.

In the modified glycoproteins with the general structure (M-L-O-N=CH)ₙ-P-(CH=N-O-L'-M')ₘ n is limited by the number of non-reducing glycan terminals of a specific glycoprotein. Thus, in one embodiment of the invention, n in the modified glycoproteins with the general structure (M-L-O-N=CH)ₙ-P-(CH=N-O-L'-M')ₘ equals the number of non-reducing glycan terminals present on the glycoprotein. In another embodiment of the invention, n in the modified glycoproteins with the general structure (M-L-O-N=CH)ₙ-P-(CH=N-O-L'-M')ₘ is in the range of 1-10, such as 1-5, such as 1-3, such as 1-2, such as 1, such as 2, such as 3.

In one embodiment the modified glycoprotein with the general structure (M-L-O-N=CH)ₙ-P-(CH=N-O-L'-M')ₘ (formula I) is selected from the list consisting of

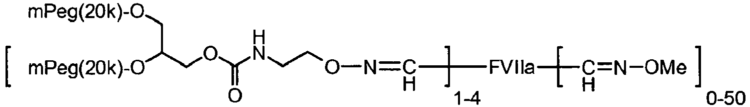

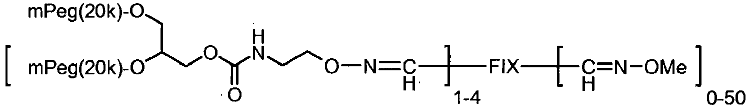

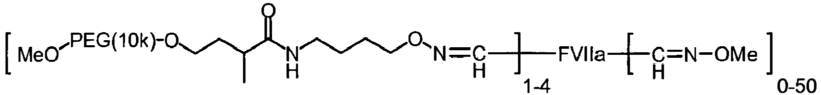

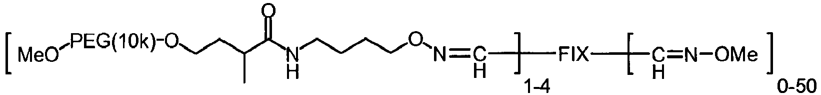

### PHARMACEUTICAL COMPOSITIONS

Another object of the present invention is to provide a pharmaceutical composition comprising a modified glycoprotein which is present in a concentration from 10⁻¹² mg/ml to 200 mg/ml, such as e.g. 10⁻¹⁰ mg/ml to 5 mg/ml and wherein said composition has a pH from 2.0 to 10.0. The composition may further comprise a buffer system, preservative(s), tonicity agent(s), chelating agent(s), stabilizers and surfactants. In one embodiment of the invention the pharmaceutical composition is an aqueous composition, i.e. composition comprising water. Such composition is typically a solution or a suspension. In a further embodiment of the invention the pharmaceutical composition is an aqueous solution. The term "aqueous composition" is defined as a composition comprising at least 50 % w/w water. Likewise, the term "aqueous solution" is defined as a solution comprising at least 50 %w/w water, and the term "aqueous suspension" is defined as a suspension comprising at least 50 %w/w water.

In another embodiment the pharmaceutical composition is a freeze-dried composition, whereto the physician or the patient adds solvents and/or diluents prior to use. In another embodiment the pharmaceutical composition is a dried composition (e.g. freeze-dried or spray-dried) ready for use without any prior dissolution.

In a further aspect the invention relates to a pharmaceutical composition comprising an aqueous solution of a Modified glycoprotein, and a buffer, wherein said Modified glycoprotein is present in a concentration from 0.1-100 mg/ml or above, and wherein said composition has a pH from about 2.0 to about 10.0.

In another embodiment of the invention the pH of the composition is selected from the list consisting of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0.

In a further embodiment of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further embodiment of the invention the composition further comprises a pharmaceutically acceptable preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomerosal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 20th edition, 2000.

In a further embodiment of the invention the composition further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic acid, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects obtained using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 20th edition, 2000.

In a further embodiment of the invention the composition further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1mg/ml to 5mg/ml. In a further embodiment of the invention the chelating agent is present in a concentration from 0.1mg/ml to 2mg/ml. In a further embodiment of the invention the chelating agent is present in a concentration from 2mg/ml to 5mg/ml. Each one of these specific chelating agents constitutes an alternative embodiment of the invention. The use of a chelating agent in pharmaceutical compositions is well-known to the skilled person. For convenience reference is made to Remington: The Science and Practice ofPharmacy, 20th edition, 2000.

In a further embodiment of the invention the composition further comprises a stabilizer. The use of a stabilizer in pharmaceutical compositions is well-known to the skilled person. For convenience reference is made to Remington: The Science and Practice of Pharmacy, 20th edition, 2000.

More particularly, compositions of the invention are stabilized liquid pharmaceutical compositions whose therapeutically active components include a protein that possibly exhibits aggregate formation during storage in liquid pharmaceutical compositions. By "aggregate formation" is intended a physical interaction between the protein molecules that results in formation of oligomers, which may remain soluble, or large visible aggregates that precipitate from the solution. By "during storage" is intended a liquid pharmaceutical composition or composition once prepared, is not immediately administered to a subject. Rather, following preparation, it is packaged for storage, either in a liquid form, in a frozen state, or in a dried form for later reconstitution into a liquid form or other form suitable for administration to a subject. By "dried form" is intended the liquid pharmaceutical composition or composition is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a protein during storage of a liquid pharmaceutical composition can adversely affect biological activity of that protein, resulting in loss of therapeutic efficacy of the pharmaceutical composition. Furthermore, aggregate formation may cause other problems such as blockage of tubing, membranes, or pumps when the protein-containing pharmaceutical composition is administered using an infusion system.

The pharmaceutical compositions of the invention may further comprise an amount of an amino acid base sufficient to decrease aggregate formation by the protein during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids to use in preparing the compositions of the invention are those carrying a charged side chain, such as arginine, lysine, aspartic acid, and glutamic acid. Any stereoisomer (i.e., L or D isomer, or mixtures thereof) of a particular amino acid (methionine, histidine, arginine, lysine, isoleucine, aspartic acid, tryptophan, threonine and mixtures thereof) or combinations of these stereoisomers or glycine or an organic base such as but not limited to imidazole, may be present in the pharmaceutical compositions of the invention so long as the particular amino acid or organic base is present either in its free base form or its salt form. In one embodiment the L-stereoisomer of an amino acid is used. In one embodiment the D-stereoisomer is used. Compositions of the invention may also be formulated with analogues of these amino acids. By "amino acid analogue" is intended a derivative of the naturally occurring amino acid that brings about the desired effect of decreasing aggregate formation by the protein during storage of the liquid pharmaceutical compositions of the invention. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the protein acting as the therapeutic agent is a protein comprising at least one methionine residue susceptible to such oxidation. By "inhibit" is intended minimal accumulation of methionine oxidized species over time. Inhibiting methionine oxidation results in greater retention of the protein in its proper molecular form. Any stereoisomer of methionine (L or D isomer) or any combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be obtained by adding methionine such that the ratio of methionine added to methionine residues ranges from about 1:1 to about 1000:1, such as 10:1 to about 100:1.

In a further embodiment of the invention the composition further comprises a stabilizer selected from the group of high molecular weight polymers or low molecular compounds. In a further embodiment of the invention the stabilizer is selected from polyethylene glycol (e.g. PEG 3350), polyvinyl alcohol (PVA), polyvinylpyrrolidone, carboxy-/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active protein therein. Stabilizing agents of particular interest to the present invention include, but are not limited to, methionine and EDTA, which protect the protein against methionine oxidation, and a nonionic surfactant, which protects the protein against aggregation associated with freeze-thawing or mechanical shearing.

In a further embodiment of the invention the composition further comprises a surfactant. In a further embodiment of the invention the surfactant is selected from a detergent, ethoxylated castor oil, polyglycolyzed glycerides, acetylated monoglycerides, sorbitan fatty acid esters, polyoxypropylene-polyoxyethylene block polymers (eg. poloxamers such as Pluronic^{®} F68, poloxamer 188 and 407, Triton X-100 ), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium tauro-dihydrofusidate etc.), long-chain fatty acids and salts thereof C₆-C₁₂ (eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulphate or sodium lauryl sulphate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-l-propanesulfonate, anionic (alkyl-aryl-sulphonates) monovalent surfactants, zwitterionic surfactants (e.g. N-alkyl-N,N-dimethylammonio-1-propanesulfonates, 3-cholamido-1-propyldimethylammonio-1-propanesulfonate, cationic surfactants (quaternary ammonium bases) (e.g. cetyl-trimethylammonium bromide, cetylpyridinium chloride), nonionic surfactants (eg. Dodecyl β-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well-known to the skilled person. For convenience reference is made to Remington: The Science and Practice of Pharmacy, 20th edition, 2000.

It is possible that other ingredients may be present in the pharmaceutical composition of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical composition of the present invention.

Pharmaceutical compositions containing a Modified glycoprotein according to the present invention may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the invention may further be compounded in, or attached to, for example through covalent, hydrophobic and electrostatic interactions, a drug carrier, drug delivery system and advanced drug delivery system in order to further enhance stability of the Modified glycoprotein, increase bioavailability, increase solubility, decrease adverse effects, achieve chronotherapy well known to those skilled in the art, and increase patient compliance or any combination thereof. Examples of carriers, drug delivery systems and advanced drug delivery systems include, but are not limited to, polymers, for example cellulose and derivatives, polysaccharides, for example dextran and derivatives, starch and derivatives, poly(vinyl alcohol), acrylate and methacrylate polymers, polylactic and polyglycolic acid and block co-polymers thereof, polyethylene glycols, carrier proteins, for example albumin, gels, for example, thermogelling systems, for example block co-polymeric systems well known to those skilled in the art, micelles, liposomes, microspheres, nanoparticulates, liquid crystals and dispersions thereof, L2 phase and dispersions there of, well known to those skilled in the art of phase behaviour in lipid-water systems, polymeric micelles, multiple emulsions, self-emulsifying, self-microemulsifying, cyclodextrins and derivatives thereof, and dendrimers.

Compositions of the current invention are useful in the composition of solids, semisolids, powder and solutions for pulmonary administration of Modified glycoprotein, using, for example a metered dose inhaler, dry powder inhaler and a nebulizer, all being devices well known to those skilled in the art.

Compositions of the current invention are specifically useful in the composition of controlled, sustained, protracting, retarded, and slow release drug delivery systems. More specifically, but not limited to, compositions are useful in composition of parenteral controlled release and sustained release systems (both systems leading to a many-fold reduction in number of administrations), well known to those skilled in the art. Even more preferably, are controlled release and sustained release systems administered subcutaneous. Without limiting the scope of the invention, examples of useful controlled release system and compositions are hydrogels, oleaginous gels, liquid crystals, polymeric micelles, microspheres, nanoparticles,

Methods to produce controlled release systems useful for compositions of the current invention include, but are not limited to, crystallization, condensation, co-crystallization, precipitation, co-precipitation, emulsification, dispersion, high pressure homogenisation, encapsulation, spray drying, microencapsulating, coacervation, phase separation, solvent evaporation to produce microspheres, extrusion and supercritical fluid processes. General reference is made to Handbook of Pharmaceutical Controlled Release (Wise, D.L., ed. Marcel Dekker, New York, 2000) and Drug and the Pharmaceutical Sciences vol. 99: Protein Composition and Delivery (MacNally, E.J., ed. Marcel Dekker, New York, 2000).
Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the Modified glycoprotein in the form of a nasal or pulmonal spray. As a still further option, the pharmaceutical compositions containing the Modified glycoprotein of the invention can also be adapted to transdermal administration, e.g. by needle-free injection or from a patch, optionally an iontophoretic patch, or transmucosal, *e.g.* buccal, administration.

The term "stabilized composition" refers to a composition with increased physical stability, increased chemical stability or increased physical and chemical stability.

The term "physical stability" of the protein composition as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein compositions is evaluated by means of visual inspection and/or turbidity measurements after exposing the composition filled in suitable containers (e.g. cartridges or vials) to mechanical/physical stress (e.g. agitation) at different temperatures for various time periods. Visual inspection of the compositions is performed in a sharp focused light with a dark background. The turbidity of the composition is characterized by a visual score ranking the degree of turbidity for instance on a scale from 0 to 3 (a composition showing no turbidity corresponds to a visual score 0, and a composition showing visual turbidity in daylight corresponds to visual score 3). A composition is classified physical unstable with respect to protein aggregation, when it shows visual turbidity in daylight. Alternatively, the turbidity of the composition can be evaluated by simple turbidity measurements well-known to the skilled person. Physical stability of the aqueous protein compositions can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.
Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like.

The term "chemical stability" of the protein composition as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein composition as well-known by the person skilled in the art. Most proteins are prone to deamidation, a process in which the side chain amide group in glutaminyl or asparaginyl residues is hydrolysed to form a free carboxylic acid. Other degradations pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein composition can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

Hence, as outlined above, a "stabilized composition" refers to a composition with increased physical stability, increased chemical stability or increased physical and chemical stability. In general, a composition must be stable during use and storage (in compliance with recommended use and storage conditions) until the expiration date is reached.
In one embodiment of the invention the pharmaceutical composition comprising the Modified glycoprotein is stable for more than 6 weeks of usage and for more than 3 years of storage.

In another embodiment of the invention the pharmaceutical composition comprising the modified glycoprotein is stable for more than 4 weeks of usage and for more than 3 years of storage.

In a further embodiment of the invention the pharmaceutical composition comprising the modified glycoprotein is stable for more than 4 weeks of usage and for more than two years of storage.

In an even further embodiment of the invention the pharmaceutical composition comprising the Modified glycoprotein is stable for more than 2 weeks of usage and for more than two years of storage.

The use of the terms "a" and "an" and "the" and similar referents in the context of describing the invention are to be construed to cover both the singular and the plural, unless otherwise indicated herein or clearly contradicted by context.

Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or embodiment of the invention using terms such as "comprising", "having," "including," or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The citation of patent documents herein is done for convenience only and does not reflect any view of the validity, patentability, and/or enforceability of such patent documents.

### EXAMPLES

### Abbreviations:

SDS-PAGE: sodium dodecyl sulphate - polyacrylamide gel electrophoresis
EDTA: ethylenediamino tetraacetic acid
FVIIa: Factor VIIa
FVIII: Factor VIII
FIX: Factor IX

### Materials, Apparatus and Methods

### Purifications:

Protein purifications on ion-exchange column (HiTrap Q HP, Amersham Bioscience) or size exclusion column (XK26/60 HiLoad Superdex 200, Amersham Bioscience) were performed using an Äkta FPLC, with a FC-950 fraction collector (Amersham Bioscience). Elution buffers, columns and fraction collector were thermostated at 5°C.

### SDS-PAGE:

SDS-PAGE was performed using Invitrogen™'s Xcell Surelock™ Mini-Cell system with precast 4-12% Bis-Tris gels, NuPAGE MES SDS running buffer, Mark 12 standard and NUPAGE LDS sample buffer according to Invitrogen's standard protocol. Gels were stained with SimpleBlue™ according to Invitrogen protocols.

### Buffer solutions

GlyGly buffer: 25 mM Gly-Gly, 50 mM NaCl, 25 mM CaCl2, pH 6.0
CaCl2 free GlyGly buffer: 25 mM Gly-Gly, 50 mM NaCl, pH 6.0
Hepes buffer: 50 mM Hepes, 100 mM NaCl, 5 mM CaCl2, 0,01% Tween 80, pH = 7.4

### Example

### NaIO₄-treatment of FVIIa and S2288 activity

Correlation study between NaIO₄ and residual peptiodlytical activity → non obvious relation.

### Assay I

### Determination of peptidolytic actitivy for FVIIa:

Peptidolytic activity was meassured using the chromogenic substrate S-2288 (*H*-D-Ile-Pro-Arg-*p*-nitroaniline) from Chromogenix, Mölndal, Sweden. The following protocol was applied: 10 mM stock solution of S2288 in Hepes buffer was prepared. Similar a 200 nM stock solution of tissue factor in Hepes buffer was prepared. 200 nM stock solutions of test entities and reference compound (e.g. wild type FVIIa) were prepared in Hepes buffer. Compounds were tested in ELISA wells in triplicates. In a typical experiment, 10 ul of 200 nM test entity solutions, 50 ul of 200 nM tissue factor stock solutions and 135 ul Hepes buffer were added to the well. The reaction was initiated by addition of 5ul of 200 nM S2288 stock solution. Wells were contineously analyzed in an ELISA - reader (absorbance at 405 nm) for 15 min. at room temperature. Relative activities were calculated from the initial rates, and compared to wild type FVIIa rates. Activities for modified FVIIa analogues were reported as a percentage of the activity of wild type FVIIa.

### Example 1

*10K PEG-ONH2:* 10K-SMB-PEG (1,00 g; 0.1 mmol; Nektar Inc) was dissolved in DCM (10 ml). 4-(*N*-t-butoxycarbonylaminoxy)butylamine (0.20 g, 1 mmol, prepared as described in WO 2005014049 A2) was added and the mixture was stirred at rt. for 16h. Diethylether (90 ml) was added and the white precipitate was filtered off. The process was repeated by redissolving the precipitate in DCM (10 ml) and adding diethylether (90 ml). The precipitated material was then redissolved in DCM (6 ml) and Amberlyst 15 ion exchange resin (2,0 g; previously washed with DCM and 10% EtOH in DCM) was added. The mixture was stirred for 30 min at rt, then the resin was filtered off, and washed extensively with DCM. The combined DCM solutions were concentrated to a minimal volumne on a rotary evaporater, then diethylether (90 ml) was added to precipitate the product. The product was dissolved in DCM (6 ml) and TFA (6 ml) was added. The mixture was stirred at rt for ½h. Product was precipitated with diethylether (100 ml), and filtered off. The product was redissolved in DCM (5 ml) and triethylamine (1 ml) was added. The mixture was stirred for 5 min, then the product was precipitated again with diethylether (100 ml). The precipitate was filtered off, washer twice with diethylether and dried overnight in vacuum oven. Yield: 475 mg (46 %). ¹H-NMR (CDCl₃; selected peaks): δ 1.25 ppm (d, 3H); 1.50-1.75 (m, 6H); 1.82 (m, 1H); 3.38 (s, 3H); 3,45-3,90 (m, ca. 900 H).

### Example 2

### mono 10K-PEG-FVIIa (0128-0000-1018-1A):

Factor VIIa (14 mg, 0.28 umol) dissolved in 10 ml 25 mM Gly-Gly, 50 mM NaCl, 25 mM CaCl2, pH 6.0 (GlyGly buffer) was added 100 ul of a 20 mM NaIO₄ solution in CaCl₂ free GlyGly buffer and 1000 ul of a solution of 10K-PEG-ONH₂ (Example 1, 42 mg; 4.2 umol; 15 eqvivalents relative to factor VIIa) in GlyGly buffer. The mixture was placed on ice for 2h, with occasional shaking. 500 ul of a solution of MeONH₂.HCl (17 mg; 0,21 mmol) in GlyGly buffer (pH adjusted to 6.0 with 1M NaOH) was then added to the reaction mixture. The mixture was left on ice for additional 10 min. The reaction mixture was then added a cold solution of 100 mM dibasic EDTA (4,5 ml) while maintaining pH below 9.0. pH was then adjusted to 8.0 using 100 ul 1N aqueous HCl.

### Ion-exchange chromatography:

Excessive PEG-ONH2 was removed by ion exchange chromatography. The cooled reaction mixture was loaded on to a 5 ml HiTrap Q ion-exchange column (Amersham Bioscience) pre-equilibrated in 25 mM GlyGly, 50 mM NaCl, pH 8.0. The column was eluded with 25 mM GlyGly, 50 mM NaCl, pH 8.0 (buffer A) over 10 cv; followed by 25 mM GlyGly, 50 mM NaCl, 25 mM CaCl₂, pH 8.0 (buffer B) over 15 cv at constant flow (1 ml/min) and temperature (5°C). The effluent was monitored by absorbance at 280 nm.

### Size exclussion chromatography:

Non pegylated FVIIa was removed from glycopegylated FVIIa variants recovered in pooled fractions from HiTrap Q by size exclusion chromatography on Superdex 200 using an ÄKTA FPLC with Frac-950 (Amersham Bioscience) run at 5°C. The XK26/60 HiLoad Superdex 200 column (320 ml cv) was pre-equilibrated (10 cv) and after loading eluted with 25 mM GlyGly, 50 mM NaCl, 25 mM CaCl2, pH 6.0, at a flow rate of 2.5 ml/min. The effluent was monitored by absorbance at 280 nm. 10 ml fractions were collected. Fractions were analyzed on SDS-PAGE (4-12% Bis-Tris NuPAGE gels) stained with Simple Blue as described by Invitrogen. Fractions containing pure mono-pegylated FVIIa were pooled and concentrated by filtering through an Amicon Ultra™-15 (10K MWCO) centrifuge filter at 4000 rpm, 10°C, for 13.5 min. obtaining a final volume of 4.1 ml.

Protein concentration was determined to be 0.29 mg/ml by UV measurements (using *E* _{280 nm} = 1.32 ml/mg/cm), which equals to 0.35 mg/ml of FVIIa-10K PEG. Peptidolytical activity (S2288 assay) was 79% relative to wt FVIIa.

### Example 3

### Preparation of high substituted (HS) and low (LS) substituted 10K-PEG-FVIIa

Factor VIIa (14 mg, 0.28 umol) dissolved in 10 ml 25 mM Gly-Gly, 50 mM NaCl, 25 mM CaCl2, pH 6.0 (GlyGly buffer) was added 100 ul of a 20 mM NaIO₄ solution in CaCl₂ free GlyGly buffer and 1000 ul of a solution of 10K-PEG-ONH₂ (Example 1, 42 mg; 4.2 umol; 15 eqvivalents relative to factor VIIa) in GlyGly buffer. The mixture was placed a 4°C for 24h, with occasional shaking. 500 ul of a solution of MeONH₂.HCl (17 mg; 0,21 mmol) in GlyGly buffer (pH adjusted to 6.0 with 1M NaOH) was then added to the reaction mixture. The mixture was left on ice for additional 10 min. The reaction mixture was then added a cold solution of 100 mM dibasic EDTA (4,5 ml) while maintaining pH below 9.0. pH was then adjusted to 8.0 using 60 ul 1N aqueous HCl.

### Ion-exchange chromatography:

Excessive PEG-ONH2 was removed by ion exchange chromatography. The cooled reaction mixture was loaded on to a 5 ml HiTrap Q ion-exchange column (Amersham Bioscience) pre-equilibrated in 25 mM GlyGly, 50 mM NaCl, pH 8.0. The column was eluded with 25 mM GlyGly, 50 mM NaCl, pH 8.0 (buffer A) over 10 cv; followed by 25 mM GlyGly, 50 mM NaCl, 25 mM CaCl₂, pH 8.0 (buffer B) over 15 cv at constant flow (1 ml/min) and temperature (5°C). The effluent was monitored by absorbance at 280 nm.

### Size exclussion chromatography:

Non pegylated FVIIa was removed from glycopegylated FVIIa variants recovered in pooled fractions from HiTrap Q by size exclusion chromatography on Superdex 200 using an ÄKTA FPLC with Frac-950 (Amersham Bioscience) run at 5°C. The XK26/60 HiLoad Superdex 200 column (320 ml cv) was pre-equilibrated (10 cv) and after loading eluted with 25 mM GlyGly, 50 mM NaCl, 25 mM CaCl2, pH 6.0, at a flow rate of 2.5 ml/min. The effluent was monitored by absorbance at 280 nm. 10 ml fractions were collected. Fractions were analyzed on SDS-PAGE (4-12% Bis-Tris NuPAGE gels) stained with Simple Blue as described by Invitrogen. Fractions were pooled according to the PEG-substitution level, and concentrated by filtering through an Amicon Ultra™-15 (10K MWCO) centrifuge filter at 4000 rpm, 10°C.
HS (tri/tetra 10K PEG) FVIIa: 0.92 mg protein (UV_{280 nm})/ml, total volume = 1.6 ml.
HS (di/tri 10K PEG) FVIIa: 0.63 mg protein (UV_{280 nm})/ml, total volume = 1.4 ml.
LS (mono/di 10K PEG) FVIIa: 1.2 mg protein (UV_{280 nm})/ml, total volume = 1.4 ml.
Mono 10K PEG FVIIa: 0.7 mg protein (UV_{280 nm})/ml, total volume = 2.3 ml.

### Example 4

### Preparation of high substituted (HS) and low (LS) substituted 40K-PEG-FVIIa

Factor VIIa (14 mg, 0.28 umol) dissolved in 15 ml 25 mM Gly-Gly, 50 mM NaCl, 25 mM CaCl2, pH 6.0 (GlyGly buffer) was added 50 ul 20 mM NaIO₄ solution in CaCl₂ free GlyGly buffer and 1500 ul of a solution of 40K-PEG-ONH₂ ((20K PEG)OCH₂(20K PEG)OCHCH₂OC(=O)O-CH₂CH₂ONH₂; WO 2006042847, 112 mg; 2.8 umol; 10 eqvivalents relative to factor VIIa) in GlyGly buffer. The mixture was placed a 4°C for 48h, with occasional shaking. 500 ul of a solution of MeONH₂.HCl (17 mg; 0,21 mmol) in GlyGly buffer was then added to the reaction mixture followed by pH adjustment to pH 6.0 using 1N NaOH. The mixture was left on ice for 10 min. The reaction mixture was then added a cold solution of 100 mM dibasic EDTA (4,5 ml) while maintaining pH below 9.0. pH was then adjusted to 8.0 using 60 ul 1N aqueous HCl.

### Ion-exchange chromatography:

Excessive PEG-ONH2 was removed by ion exchange chromatography. The cooled reaction mixture was loaded on to a 5 ml HiTrap Q ion-exchange column (Amersham Bioscience) pre-equilibrated in 25 mM GlyGly, 50 mM NaCl, pH 8.0. The column was eluded with 25 mM GlyGly, 50 mM NaCl, pH 8.0 (buffer A) over 10 cv; followed by 25 mM GlyGly, 50 mM NaCl, 25 mM CaCl₂, pH 8.0 (buffer B) over 15 cv at constant flow (1 ml/min) and temperature (5°C). The effluent was monitored by absorbance at 280 nm. Fractions were pooled and pH adjusted to 6.0 using1N HCl.

### Size exclussion chromatography:

Non pegylated FVIIa was removed from glycopegylated FVIIa variants recovered in pooled fractions from HiTrap Q by size exclusion chromatography on Superdex 200 using an ÄKTA FPLC with Frac-950 (Amersham Bioscience) run at 5°C. The XK26/60 HiLoad Superdex 200 column (320 ml cv) was pre-equilibrated (10 cv) and after loading eluted with 25 mM GlyGly, 50 mM NaCl, 25 mM CaCl2, pH 6.0, at a flow rate of 2.5 ml/min. The effluent was monitored by absorbance at 280 nm. 10 ml fractions were collected. Fractions containing UV activity (280 nm) were analyzed on SDS-PAGE (4-12% Bis-Tris NuPAGE gels) stained with Simple Blue as described by Invitrogen. Fractions were pooled according to the PEG-substitution level, and concentrated by filtering through an Amicon Ultra™-15 (10K MWCO) centrifuge filter at 4000 rpm, 10°C.
HS (di/tri 40K PEG) FVIIa: 0.46 mg protein (UV_{280 nm})/ml, total volume = 3.0 ml.
LS (mono/di 40K PEG) FVIIa: 0.75 mg protein (UV_{280 nm})/ml, total volume = 3.7 ml.
Mono 40K-PEGFVIIa: 0.53 mg protein (UV_{280 nm})/ml, total volume = 4.5 ml.

### Example 5

### Preparation of high substituted (HS) and low (LS) substituted 5K-PEG-FVIIa

### - Influence on periodate concentration and peptidolytical activity of the final compound.

The following stock solutions were prepared:
2.8 mM PEG stock solution: 5K-PEG-ONH₂ (4.03 mg; 0.8 umol, prepared from 5K-PEG-NHS ester as described in Example 1) dissolved in 281 ul CaCl₂ free GlyGly buffer (25 mM Gly-Gly, 50 mM NaCl, pH 6.0). 20 mM NaIO₄ stock solution: NaIO₄ (426 mg; 2 mmol) in 100 ml CaCl₂ free GlyGly buffer (25 mM Gly-Gly, 50 mM NaCl, pH 6.0). 100 mM EDTA solution: EDTA - disodium salt (292 mg) in 10 ml water. 1M CaCl₂-solution: CaCl2 (1.12g) in 10 ml water (filtered through 0.45 um filter). 12.8 uM Factor VIIa solution: (1.4 mg, 0.028 umol) dissolved in 1.0 ml 25 mM Gly-Gly, 50 mM NaCl, 25 mM CaCl₂, pH 6.0 (GlyGly buffer) was added 95 ul of a 100 mM EDTA solution followed by 1.095 ml CaCl₂ free GlyGly buffer (final FVIIa concentration = 0.64 mg/ml). Stock solutions were mixed according to the following scheme:

| Entry | 12.8 uM FVIIa | CaCl₂-free GlyGly buffer | 20 mM NaIO₄ | 2.8 mM PEG5000-ONH2 | total volume |
|---|---|---|---|---|---|
| 1 | 50 ul | 40 ul | 0 ul | 10 ul | 100 ul |
| 2 | 50 ul | 20 ul | 20 ul | 10 ul | 100 ul |
| 3 | 50 ul | 30 ul | 10 ul | 10 ul | 100 ul |
| 4 | 50 ul | 38 ul | 2 ul | 10 ul | 100 ul |
| 5 | 50 ul | 39,5 ul | 0,5 ul | 10 ul | 100 ul |

All the reaction mixtures was shaken gently at room temperature for 2h. To each vial was then added 25 ul 1M CaCl₂ solution. Samples were then analyzed for peptiodlytical activity as described in the assay section.

| Entry | Final NaIO₄-conc. | FVIIa:NaIO4 ratio | Sialic Acid :NaIO₄ ratio* | Relative peptidolytical activity |
|---|---|---|---|---|
| 1 | 0.0 mM | 1:0 | 1:0 | 100 |
| 2 | 4.0 mM | 1:625 | 1:138 | 3,6 |
| 3 | 2.0 mM | ∼1:312 | ∼1:70 | 15 |
| 4 | 0.4 mM | ∼1:63 | ∼1:14 | 47 |
| 5 | 0.1 mM | ∼1:16 | ∼1:3.5 | 82 |

| | | | | |
|---|---|---|---|---|
| * Average number of sialic acids on FVIIa equals 4.5 as determined by neuraminidase - galactose oxidase assay as described in example 8 below. | | | | |

In all cases, an indetical mixture of mono- di- and tri pegylated product was obtained, as analyzed by SDS-PAGE (4-12% Bis-Tris NuPAGE gels) stained with SilverQuest as described by Invitrogen.

### Example 6:

### Preparation of high substituted (HS) and low (LS) substituted 10K-PEG-FIX:

These materials are prepared using the protocol described in example 3.

### Example 7:

### Preparation of high substituted (HS) and low (LS) substituted 40K-PEG-FIX

Factor IX (BeneFix) was purified from excipients according to the following procedure: The freeze dried material (1000 IU) was reconstituted in sterile water (4 ml) and added EDTA (200 ul, 0.25M aqueous solution, pH 6). After 10 min at 5 °C, the sample was loaded un to a 1 ml Resource Q column (Amersham Bioscience) pre-equilibrated in 25 mM GlyGly, 100 mM NaCl, pH 6.0. The column was operated at a flow rate of 1 ml/min. The column was eluded with 25 mM GlyGly, 100 mM NaCl, pH 6.0 (buffer A) over 30 cv; followed by 25 mM GlyGly, 100 mM NaCl, 10 mM CaCl₂, pH 6.0 (buffer B) over 15 cv at constant flow (1 ml/min) and temperature (5°C). The effluent was monitored by absorbance at 280 nm. A 4.2 ml fraction was collected, and protein concentration was determined by absorbance (1.32/mg/ml) to be 0.52 mg/ml.

### Pegylation reaction:

Purified Benefix in 25 mM GlyGly, 100 mM NaCl, pH 6.0 (2 ml; 1.04 mg; 18.54 mmol) was added 80 ul (1 mM NaIO4, 80 nmol, 1 equivalent relative to sialic acid content on BeneFix). The mixture was stirred at room temperature for 2h, then a solution of 40K-PEG-ONH₂ ((20K PEG)OCH₂(20K PEG)OCHCH₂OC(=O)O-CH₂CH₂ONH₂; WO 2006042847; 926 ul; 500 uM; 25x) in 25 mM GlyGly, 100 mM NaCl, pH 6.0 was added. The mixture was left at rt for 48h. Metheonine (100 ul, 5mM in 25 mM GlyGly, 100 mM NaCl, pH 6.0), followed be MeONH2 (100 ul, 5 mM in 25 mM GlyGly, 100 mM NaCl, pH 6.0) was then added. The mixture was left at room temperature for 30 min, and then frozen to -80 °C until further purification.

### Ion-exchange chromatography:

Excessive PEG-ONH2 reagent, and non pegylated FIX was removed from the glycopegylated FIX variants by ion-exchange chromatography using a 1 ml Resource Q column operated on an ÄKTA FPLC with Frac-950 (all from Amersham Bioscience). The effluent was monitored by absorption at 280 nm. 1000 ul fractions were collected, and the flow was maintained at 1 ml/min during the process. The frozen reaction mixture was slowly thawed, and added EDTA (400 ul, 0.25M aqueous solution, pH 6). Conductivity of the mixture was adjusted to 9.3 mS by addition of water. The mixture was then applied on to the Resource Q column which was pre-equilibrated in 20 cv of 10 mM histidine, 50 mM NaCl, 10 mM EDTA, 0.01% Tween 80, pH 6.0. The column was washed with 15 cv of 10 mM histidine, 50 mM NaCl, 10 mM EDTA, 0.01% Tween 80, pH 6.0 followed by 15 cv of 10 mM histidine, 50 mM NaCl, 0.01% Tween 80, pH 6.0 (buffer A). The column was then eluted with an increasing gradient (0-80%) of 10 mM histidine, 50 mM MgCl2, 0.01% Tween 80, pH 6.0 (buffer B), over 15 cv, followed by 100% buffer B over 20 cv. Fractions were analyzed on SDS-PAGE (4-12% Bis-Tris NuPAGE gels) stained with Simple Blue as described by Invitrogen. Fractions were pooled according to the PEG-substitution level.
LS (mono/di 40K PEG) FIX: 34.3 ug protein (UV_{280 nm})/ml, total volume = 3 ml.
Mono 40K-PEG-FIX: 70.7 ug protein (UV_{280 nm})/ml, total volume = 3 ml.

### Example 8:

### Quantification of sialic acids content of FVIIa:

Sialic acid content was determined using Amplex Red Neuraminidase (Sialidase) Assay Kit (A-22178) from Molecular Probes Inc. (29851 Willow Creek Road, Eugene, OR 97402), as described in WO 2005014035 A2.

### Example 9:

### Periodate mediated 40K-pegylation of FVIII

Factor VIII (Refacto; Wyeth, 2000 IE) was dissolved in 1 ml dispension buffer containing: NaCl (36 mg/ml); saccharose (12 mg/ml); L-histidine (6 mg/ml); KCI (1 mg/ml); polysorbat 80 (0,4 mg/ml). Then 400 IE (in 200 ul constitution buffer) was bufferexchanged into 20 mM GlyGly, 0.15 M NaCl; 10 mM CaCl2; 0.02% Tween 80, pH 7.3 by spinfiltration using Amicon Ultra 15, 10K MWCO (3x12 min, 13.000 rpm). To 30 ul of this FVIII solution was added 10 ul of a 500 uM 40K-PEG-ONH2 (SunBright GL2-400CA; 20 mg/ml) in 20 mM GlyGly, 0.15 M NaCl; 10 mM CaCl2; 0.02% Tween 80, pH 7.3 followed by 3 ul of a 100 uM aqueous NaIO₄ and 12 ul 20 mM GlyGly, 0.15 M NaCl; 10 mM CaCl2; 0.02% Tween 80, pH 7.3 solution. Reaction was incubated for 16h@4°C, then analyzed by SDS-Gel, (7% Tris acetat gel 150V/70 min) and silver stained with SilverQuest as described by Invitrogen.

### EXEMPLARY CLAUSES

To better illustrate the invention described herein, a nonlimiting list of some of exemplary clauses and features of the invention are provided here:
1. A method for preparing a modified glycoprotein with the general structure

   (M-L-O-N=CH)ₙ-P-(CH=N-O-L'-M')ₘ (formula I),

   wherein M and optionally M' independently is a polymeric moiety for increasing the molecular weight of the modified glycoprotein, and wherein L and L' independently represent a bivalent linker, and wherein P represents a glycoprotein comprising one or more oxidized glucan terminals of said glycoprotein, O, N, C and H represents oxygen, nitrogen, carbon and hydrogen atoms respectively, n is 1-10, m is 0-50, the method comprising the steps of
   a) oxidizing with periodate ions at least one glycan terminal present on glycoprotein P*, wherein P* represents a plurality of glycoforms to obtain the glycoprotein P-(CHO)ₙ₊ₘ containing one or more aldehyde groups, and
   b) reacting P(CHO)ₙ₊ₘ with M-L-O-NH₂ to obtain the modified glycoprotein with the structure (M-L-O-N=CH)ₙ-P-(CHO)ₘ, and
   c) reacting any non-reacted aldehyde group in glycoprotein with structure (M-L-O-N=CH)ₙ-P-(CHO)ₘ with M'-L'-O-NH₂ to obtain the modified glycoprotein with the structure (M-L-O-N=CH)ₙ-P-(CH=N-O-L'-M')ₘ,
   wherein said periodate ions are present in an amount of 0.1-5 equivalents relative to the number of non-reducing glycan terminals present on the glycoprotein, and wherein said modified glycoprotein has improved pharmacologic properties compared to the starting glycoprotein P* and has retained its functional activity.
2. The method according to embodiment 1, wherein said glycoprotein is N-glycosylated and/or O-glycosylated and/or contains sialic acid moieties.
3. The method according to any one of the preceding embodiments, which comprises the further step of confirming that the modified glycoprotein has improved pharmacologic properties compared to the starting glycoprotein.
4. The method according to any one of the preceding embodiments, wherein the improved pharmacologic property is selected from the group consisting of increased bioavailability, increased functional *in vivo* half-life, increased *in vivo* plasma half-life, reduced immunogenicity, increased protease resistance, increased affinity for albumin, improved affinity for a receptor, increased storage stability, decreased functional *in vivo* half-life, decreased *in vivo* plasma half-life.
5. The method according to embodiment 4, wherein the increased half-life is obtained by M and/or M' being a group that increases molecular weight so that renal clearance is reduced or abolished and/or by M being a group that masks binding partners for hepatic receptors.
6. The method according to embodiment 4, wherein the reduced immunogenicity is obtained by M and/or M' being a group which blocks antibody binding to immunogenic sites.
7. The method according to embodiment 4, wherein the improved affinity for albumin is obtained by M and/or M' being a group which has high affinity for albumin.
8. The method according to embodiment 4, wherein the improved affinity for a receptor is obtained by M and/or M' being a group which specifically binds a surface receptor on a target cell.
9. The method according to any one of the preceding embodiments, wherein M and/or M' is selected from the group consisting of: a low molecular weight organic charged radical, which may contain one or more carboxylic acids, amines, sulfonic acids, phosphonic acids, or combinations thereof; a low molecular weight neutral hydrophilic molecule, such as cyclodextrin or a optionally branched polyethylene chain; a low molecular weight hydrophobic molecule such as a fatty acid or cholic acid or derivatives thereof; a polyethylene glycol with an average molecular weight of 2-40 kDa; a well-defined precision polymer such as a dendrimer with an exact molecular mass ranging from 700 Da to 20 kDa; a substantially non-immunogenic polypeptide such as albumin, an antibody or a part of an antibody optionally containing a Fc-domain; and a high molecular weight organic polymer.
10. The method according to any to any one of the preceding embodiments, wherein M and/or M' is selected from the group consisting of a dendrimer, polyalkylene oxide (PAO), including polyalkylene glycol (PAG), such as polyethylene glycol (PEG) and polypropylene glycol (PPG), branched PEG, polyvinyl alcohol (PVA), polycarboxylate, poly-vinylpyrolidone, polyethylene-co-maleic acid anhydride, polystyrene-co-maleic acid anhydride, dextran, carboxymethyl-dextran, HES, MPC, and PHF.
11. The method according to any one of embodiments 1-9, wherein M and/or M' is selected from the group consisting of a serum protein binding-ligand and a small organic molecule containing moieties that under physiological conditions alters charge properties, a structure which inhibits glycans from binding to receptors, and a neutral substituent that prevent glycan specific recognition.
12. The method according to any one of the preceding embodiments, wherein P is selected from FVII, FVIII, FIX, FX, FII, FV, protein C, protein S, tPA, PAI-1, tissue factor, FXI, FXII, FXIII, as well as sequence variants thereof; immunoglobulins, cytokines such as interleukins, alpha-, beta-, and gamma-interferons, colony stimulating factors including granulocyte colony stimulating factors, fibroblast growth factors, platelet derived growth factors, phospholipase-activating protein (PUP), insulin, plant proteins such as lectins and ricins, tumor necrosis factors and related alleles, soluble forms of tumor necrosis factor receptors, interleukin receptors and soluble forms of interleukin receptors, growth factors such as tissue growth factors, such as TGFa's or TGFps and epidermal growth factors, hormones, somatomedins, erythropoietin, pigmentary hormones, hypothalamic releasing factors, antidiuretic hormones, prolactin, chorionic gonadotropin, follicle-stimulating hormone, thyroid-stimulating hormone, tissue plasminogen activator, and immunoglobulins such as IgG, IgE, IgM, IgA, and IgD, and fragments thereof, or any fusion proteins comprising any of the above mentioned proteins or fragments thereof.
13. The method according to any one of embodiments 1-12, wherein the glycoprotein is a Factor VII polypeptide.
14. The method according to any one of embodiments 1-12, wherein the glycoprotein has the amino acid sequence of wild-type human Factor VII.
15. The method according to any one of embodiments 13-14, wherein the modified glycoprotein exhibit at least about 10 %, such as at least about 20 %, such as at least about 40 %, such as at least about 60 %, such as at least about 80 %, such as at least about 100 % of the specific activity of un-modified Factor VII polypeptide when tested in one or more of a clotting assay, proteolysis assay, or TF binding assay as described in the present specification.
16. The method according to any one of embodiments 1-15, wherein the modified glycoprotein exhibits a bioavailability that is at least about 110% of the bioavailability of the un-modified glycoprotein, such as at least about 120%, about 130%, or at least about 140% of the bioavailability of the un-modified glycoprotein.
17. The method according to any one of embodiments 1-15, wherein the modified glycoprotein exhibits a serum half-life that is at least about 125% of the half-life of the unmodified glycoprotein, such as about 150%, about 200%, or at least about 250% of the half-life of the un-modified glycoprotein.
18. The method according to any one of embodiments 1-17, wherein said periodate ions are present in an amount of less than 20 equivalents, such as less than 10 equivalents, such as less than 5 equivalents, such as less than 1 equivalents relative to the number of non-reducing glycan terminals present on the glycoprotein, such as in the range of 0.1-20 equivalents, such as in the range of 0.1-10 equivalents, such as in the range of 0.1-5 equivalents, such as in the range of 0.1-1 equivalents, relative to the number of non-reducing glycan terminals present on the glycoprotein.

## Claims

1. A method for preparing a modified glycoprotein with the general structure (M-L-O-N=CH)ₙ-P-(CH=N-O-L'-M')ₘ (formula I)
wherein M and optionally M' independently is a polymeric moiety for increasing the molecular weight of the modified glycoprotein, and wherein L and L' independently represent a bivalent linker, and wherein P represents a glycoprotein comprising one or more oxidized glucan terminals of said glycoprotein, O, N, C and H represents oxygen, nitrogen, carbon and hydrogen atoms respectively, n is 1-10, m is 0-50, the method comprising the steps of
a) oxidizing with periodate ions at least one glycan terminal present on glycoprotein P*, wherein P* represents a plurality of glycoforms to obtain the glycoprotein P-(CHO)ₙ₊ₘ containing one or more aldehyde groups, and
b) reacting P(CHO)ₙ₊ₘ with M-L-O-NH₂ to obtain the modified glycoprotein with the structure (M-L-O-N=CH)ₙ-P-(CHO)ₘ, and
c) reacting any non-reacted aldehyde group in glycoprotein with structure (M-L-O-N=CH)ₙ-P-(CHO)ₘ with M'-L'-O-NH₂ to obtain the modified glycoprotein with the structure (M-L-O-N=CH)ₙ-P-(CH=N-O-L'-M')ₘ,
wherein said periodate ions are present in an amount of 0.1-5 equivalents relative to the number of non-reducing glycan terminals present on the glycoprotein, and wherein said modified glycoprotein has improved pharmacologic properties compared to the starting glycoprotein P* and has retained its functional activity, and wherein P is selected from FVII, FVIII and FIX.

2. The method according to claim 1, wherein M and/or M' is selected from the group consisting of: a low molecular weight organic charged radical, which may contain one or more carboxylic acids, amines, sulfonic acids, phosphonic acids, or combinations thereof; a low molecular weight neutral hydrophilic molecule, such as cyclodextrin or a optionally branched polyethylene chain; a low molecular weight hydrophobic molecule such as a fatty acid or cholic acid or derivatives thereof; a polyethylene glycol with an average molecular weight of 2-40 kDa; a well-defined precision polymer such as a dendrimer with an exact molecular mass ranging from 700 Da to 20 kDa; a substantially non-immunogenic polypeptide such as albumin, an antibody or a part of an antibody optionally containing a Fc-domain; and a high molecular weight organic polymer.

3. The method according to any one of the preceding claims, wherein M and/or M' is selected from the group consisting of a dendrimer, polyalkylene oxide (PAO), including polyalkylene glycol (PAG), such as polyethylene glycol (PEG) and polypropylene glycol (PPG), branched PEG, polyvinyl alcohol (PVA), polycarboxylate, poly-vinylpyrolidone, polyethylene-co-maleic acid anhydride, polystyrene-co-maleic acid anhydride, dextran, carboxymethyldextran, HES, MPC, and PHF.

4. The method according to any one of claims 1-3, wherein the glycoprotein is a Factor VII polypeptide.

5. The method according to any one of claims 1-4, wherein the modified glycoprotein exhibits a bioavailability that is at least about 110% of the bioavailability of the un-modified glycoprotein, such as at least about 120%, about 130%, or at least about 140% of the bioavailability of the un-modified glycoprotein.

6. The method according to any one of claims 1-5 , wherein said periodate ions are present in an amount of 0.1-1 equivalents, relative to the number of non-reducing glycan terminals present on the glycoprotein.

## Patentansprüche

1. Verfahren zur Herstellung eines modifizierten Glykoproteins mit der allgemeinen Struktur (M-L-O-N=CH)ₙ-P-(CH=N-O-L'-M')ₘ (Formel I),
wobei M und gegebenenfalls M' unabhängig voneinander für eine polymere Gruppierung zum Erhöhen des Molekulargewichts des modifizierten Glykoproteins stehen und wobei L und L' unabhängig voneinander für ein bivalentes Brückenglied stehen und wobei P für ein Glykoprotein steht, welches einen oder mehrere oxidierte Glucantermini des Glykoproteins umfasst, O, N, C und H für Sauerstoff-, Stickstoff-, Kohlenstoffbeziehungsweise Wasserstoffatome stehen, n für 1-10 steht, m für 0-50 steht, wobei das Verfahren die folgenden Schritte umfasst:
a) Oxidieren mindestens eines an dem Glykoprotein P* vorhandenen Glycanterminus mit Periodationen, wobei P* für eine Mehrzahl von Glykoformen steht, unter Erhalt des Glykoproteins P-(CHO)ₙ₊ₘ mit einer oder mehreren Aldehydgruppen, und
b) Umsetzen von P(CHO)ₙ₊ₘ mit M-L-O-NH₂ unter Erhalt des modifizierten Glykoproteins mit der Struktur (M-L-O-N=CH)ₙ-P-(CHO)ₘ, und
c) Umsetzen von jeglichen nicht umgesetzten Aldehydgruppen in Glykoprotein mit der Struktur (M-L-O-N=CH)ₙ-P-(CHO)ₘ mit M'-L'-O-NH₂ unter Erhalt des modifizierten Glykoproteins mit der Struktur (M-L-O-N=CH)ₙ-P-(CH=N-O-L'-M')ₘ,
wobei die Periodationen in einer Menge von 0,1-5 Äquivalenten bezogen auf die Anzahl von an dem Glykoprotein vorhandenen nicht reduzierenden Glycantermini vorhanden sind, und wobei das modifizierte Glykoprotein im Vergleich zum AusgangsGlykoprotein P* verbesserte pharmakologische Eigenschaften unter Beibehaltung seiner funktionellen Aktivität aufweist, und wobei P aus FVII, FVIII und FIX ausgewählt ist.

2. Verfahren nach Anspruch 1, wobei M und/oder M' ausgewählt ist aus der Gruppe bestehend aus: einem niedermolekularen organischen geladenen Rest, welcher eine oder mehrere Carbonsäuren, Amine, Sulfonsäuren, Phosphonsäuren oder Kombinationen davon enthalten kann; einem niedermolekularen neutralen hydrophilen Molekül wie Cyclodextrin oder einer gegebenenfalls verzweigten Polyethylenkette; einem niedermolekularen hydrophoben Molekül wie einer Fettsäure oder Cholsäure oder Derivaten davon; einem Polyethylenglycol mit einem durchschnittlichen Molekulargewicht von 2-40 kDa; einem gut definierten Präzisionspolymer wie einem Dendrimer mit einer exakten Molekülmasse im Bereich von 700 Da bis 20 kDa; einem im Wesentlichen nicht immunogenen Polypeptid wie Albumin, einem Antikörper oder einem Teil eines Antikörpers, gegebenenfalls mit einer Fc-Domäne; und einem hochmolekularen organischen Polymer.

3. Verfahren nach einem der vorhergehenden Ansprüche, wobei M und/oder M' ausgewählt ist aus der Gruppe bestehend aus: einem Dendrimer, Polyalkylenoxid (PAO) einschließlich Polyalkylenglycol (PAG) wie Polyethylenglycol (PEG) und Polypropylenglycol (PPG), verzweigtem PEG, Polyvinylalkohl (PVA), Polycarbonat, Polyvinylpyrrolidon, Polyethylen-co-maleinsäure-anhydrid, Polystyrol-co-maleinsäureanhydrid, Dextran, Carboxymethyldextran, HES, MPC und PHF.

4. Verfahren nach einem der Ansprüche 1-3, wobei es sich bei dem Glykoprotein um ein Faktor-VII-Polypeptid handelt.

5. Verfahren nach einem der Ansprüche 1-4, wobei das modifizierte Glykoprotein eine Bioverfügbarkeit aufweist, die mindestens etwa 110% der Bioverfügbarkeit des nichtmodifizierten Glykoproteins beträgt, wie mindestens etwa 120%, etwa 130% oder mindestens etwa 140% der Bioverfügbarkeit des nichtmodifizierten Glykoproteins.

6. Verfahren nach einem der Ansprüche 1-5, wobei die Periodationen in einer Menge von 0,1-1 Äquivalenten bezogen auf die Anzahl von an dem Glykoprotein vorhandenen nichtreduzierenden Glycantermini vorhanden sind.

## Revendications

1. Procédé de préparation d'une glycoprotéine modifiée ayant la structure générale (M-L-O-N=CH)ₙ-P-(CH=N-O-L'-M')ₘ (formule I)
dans laquelle M et facultativement M' est indépendamment un fragment polymère pour augmenter le poids moléculaire de la glycoprotéine modifiée, et dans laquelle L et L' représentent indépendamment un lieur bivalent, et dans laquelle P représente une glycoprotéine comprenant une ou plusieurs terminaisons glucane oxydées de ladite glycoprotéine, O, N, C et H représentent des atomes d'oxygène, d'azote, de carbone et d'hydrogène, respectivement, n est 1 à 10, m est 0 à 50, le procédé comprenant les étapes de
a) oxydation avec des ions periodate d'au moins une terminaison glycane présente sur la glycoprotéine P*, où P* représente une pluralité de glycoformes pour obtenir la glycoprotéine P-(CHO)ₙ₊ₘ contenant un ou plusieurs groupes aldéhyde, et
b) réaction de P(CHO)ₙ₊ₘ avec M-L-O-NH₂ pour obtenir la glycoprotéine modifiée ayant la structure (M-L-O-N=CH)ₙ-P-(CHO)ₘ, et
c) réaction des groupes aldéhyde n'ayant pas réagi éventuels dans la glycoprotéine ayant la structure (M-L-O-N=CH)ₙ-P-(CHO)ₘ avec M'-L'-O-NH, pour obtenir la glycoprotéine modifiée ayant la structure (M-L-O-N=CH)ₙ-P-(CH=N-O-L'-M')ₘ,
dans lequel lesdits ions periodate sont présents en une quantité de 0,1 à 5 équivalents par rapport au nombre de terminaisons glycane réductrices présentes sur la glycoprotéine, et dans lequel ladite glycoprotéine modifiée a des propriétés pharmacologiques améliorées par rapport à la glycoprotéine de départ P* et a conservé son activité fonctionnelle, et dans lequel P est choisi parmi FVII, FVIII et FIX.

2. Procédé selon la revendication 1, dans lequel M et/ou M' est choisi dans le groupe constitué de : un radical chargé organique de faible poids moléculaire, qui peut contenir un ou plusieurs acides carboxyliques, amines, acides sulfoniques, acides phosphoniques, ou des combinaisons de ceux-ci ; une molécule hydrophile neutre de faible poids moléculaire, telle que la cyclodextrine ou une chaîne de polyéthylène facultativement ramifiée ; une molécule hydrophobe de faible poids moléculaire telle qu'un acide gras ou l'acide cholique ou des dérivés de ceux-ci ; un polyéthylène glycol ayant un poids moléculaire de 2 à 40 kDa ; un polymère de précision bien défini tel qu'un dendrimère ayant une masse moléculaire exacte dans la plage de 700 Da à 20 kDa ; un polypeptide sensiblement non-immunogène tel que l'albumine, un anticorps ou une partie d'un anticorps contenant facultativement un domaine Fc ; et un polymère organique de poids moléculaire élevé.

3. Procédé selon l'une quelconque des revendications précédentes, dans lequel M et/ou M' est choisi dans le groupe constitué d'un dendrimère, un oxyde de polyalkylène (PAO), comprenant un polyalkylène glycol (PAG) tel que le polyéthylène glycol (PEG) et le polypropylèneglycol (PPG), un PEG ramifié, l'alcool polyvinylique (PVA), le polycarboxylate, la polyvinylpyrolidone, un copolymère de polyéthylène-anhydride d'acide maléique, un copolymère de polystyrène-anhydride d'acide maléique, le dextrane, le carboxyméthyl-dextrane, HES, MPC, et PHF.

4. Procédé selon l'une quelconque des revendications 1 à 3, dans lequel la glycoprotéine est un polypeptide de facteurs VII.

5. Procédé selon l'une quelconque des revendications 1 à 4, dans lequel la glycoprotéine modifiée présente une biodisponibilité qui est au moins environ 110 % de la biodisponibilité de la glycoprotéine non modifiée, par exemple au moins environ 120 %, environ 130 %, ou au moins environ 140 % de la biodisponibilité de la glycoprotéine non modifiée.

6. Procédé selon l'une quelconque des revendications 1 à 5, dans lequel lesdits ions periodate sont présents en une quantité de 0,1 à 1 équivalent, par rapport au nombre de terminaisons glycane non réductrices présentes dans la glycoprotéine.
